# EUROPEAN PATENT SPECIFICATION

(11) **EP 4 065 733 B1**
(45) Date of publication and mention of the grant of the patent: **01.05.2024**
(21) Application number: 20845245.8
(22) Date of filing: 26.11.2020
(51) Int. Cl.: C12Q 1/689

(54) **METHOD AND KIT FOR DETECTION OF EXTRAINTESTINAL E. COLI STRAINS PATHOGENIC TO POULTRY**
VERFAHREN UND KIT ZUR DETEKTION VON EXTRAINTESTINALEN E. COLISTÄMMEN, DIE FÜR GEFLÜGEL PATHOGEN SIND
PROCÉDÉ ET KIT POUR LA DÉTECTION DE SOUCHES D'E. COLI EXTRA-INTESTINALES PATHOGÈNES POUR LA VOLAILLE

(30) Priority: 26.11.2019 PL 43194219
(43) Date of publication of application: 05.10.2022
(73) Proprietor: Proteon Pharmaceuticals S.A., 90-364 Lódz (PL)
(72) Inventor: DASTYCH, Jaroslaw, 92-009 Lódz (PL); KAZIMIERCZAK, Joanna, 92-417 Lódz (PL); POSPIECH, Karolina, 94-121 Lódz (PL); SOWINSKA, Patrycja, 94-102 Lódz (PL); WÓJCIK, Ewelina A., 97-306 Grabica (PL); STANCZYK, Malgorzata, 91-157 Lódz (PL); ANDRYSIAK, Justyna, 92-413 Lódz (PL); STRAPAGIEL, Dominik, 92-223 Lódz (PL); MARCINIAK, Blazej, 93-504 Lódz (PL); BORÓWKA, Paulina, 92-314 Lódz (PL); LIS, Marcin Wojciech, 32-020 Wieliczka (PL)
(74) Representative: Witek, Rafal
(86) International application number: PCT/PL2020/050089
(87) International publication number: WO 2021/107795

(56) References cited:
- US-A1- 2002 102 546
- T. J. JOHNSON ET AL: "Identification of Minimal Predictors of Avian Pathogenic Escherichia coli Virulence for Use as a Rapid Diagnostic Tool", JOURNAL OF CLINICAL MICROBIOLOGY, vol. 46, no. 12, 1 December 2008 (2008-12-01), pages 3987-3996, XP055659702, US ISSN: 0095-1137, DOI: 10.1128/JCM.00816-08
- VANDEKERCHOVE D ET AL: "Virulence-associated traits in avian Escherichia coli: Comparison between isolates from colibacillosis-affected and clinically healthy layer flocks", VETERINARY MICROBIOLOGY, ELSEVIER BV, NL, vol. 108, no. 1-2, 15 June 2005 (2005-06-15), pages 75-87, XP027620543, ISSN: 0378-1135 [retrieved on 2005-06-15]

## Description

The subject of the invention is a rapid method allowing the unambiguous identification of extra intestinal strains of *E. coli* pathogenic to poultry (APEC - Avian Pathogenic *Escherichia coli),* by means of a new combination of genes amplified in the multiplex PCR diagnostic method, Pathogenic *E. coli* causes colibacillosis, a common disease of domestic birds, which is an important issue in the safety of animal breeding, and is one of the main causes of high poultry mortality and the related significant economic losses in the poultry industry (Barnes, HJ. Et al., 2008, Ewers, C. et al., 2008, Lutful Kabir, SM., 2010). So far, it has been found that APEC strains have many similarities with other extraintestinal strains of *E. coli* (ExPEC - Extraintestinal pathogenic *Escherichia coli)* pathogenic for humans, including UPEC (uropathogenic *E. coli*), NMEC (neonatal meningitis-associated *E. coli*) and SEPEC (sepsis-causing *E. coli*) strains and pose a potential risk to human health (Mellata, M., 2013).

Pathogenic *E. coli* strains are resistant to many antimicrobial agents, therefore commonly used antibiotic therapies are generally ineffective. So far, no effective vaccine has been developed against these bacteria (Huja, S. et al., 2015). More and more evidence also points to the fact that some APECs are zoonotic pathogens (Hussein A.H., 2013) and the number of infections caused by these bacteria is constantly increasing (Dziva, F. et al., 2008).

A common problem with APEC strains is the lack of a unified identification system. This is due to several factors:
- the prevalence of *E. coli* in the environment of animals: *E. coli* is present in a commensal microflora that can be a reservoir of pathogenicity without causing any pathogenic effect; additionally, it is difficult to isolate only pathogenic strains from the organs of sick birds (Lindstedt, BA. et al., 2018, Stromberg, ZR. et al., 2017, Sarowska, J. et al., 2019);
- the multiplicity of serotypes among *E. coli* strains - there are serotypes that can be associated with pathogenicity (e.g. 01, 02, 078), however many strains cannot be typed by traditional serological methods or there are rare serotypes that cannot be linked to the pathogenicity of the strains (Dziva, F. et al., 2012, Jorgensen, SL et al., 2017, Iguchi, A. et al., 2015);
- huge genetic diversity - extraintestinal strains of *E. coli* possess mechanisms that determine their pathogenicity and adapt them to life in the extraintestinal environment; virulence factors are mainly related to adhesion, toxin production and iron harvesting mechanisms, however there is no common method for determining which factors have a direct impact on pathogenicity and there is no minimal number of such factors that is considered to be a good predictor of pathogenicity. There are many studies that indicate the presence of certain selected virulence factors in pathogenic strains, however, the statistical significance of the occurrence of the analyzed genes in pathogenic strains in relation to non-pathogenic strains has not been established (Dissanayake, DR. et al., 2013, Subedi, M. et al. , 2018, Silveira, F. et al., 2016, Johnson, TJ. Et al., 2008, Huja, S. et al., 2015, Guabiraba R. & Schouler, C., 2015, Paixao, AC et al., 2016).

Currently, the most commonly used diagnostic method for typing APEC strains is serological identification. However, this method allows for the identification of only limited number of strains and does not reflect the presence of virulence factors (Schouler, C. et al., 2012).

Therefore, without the precise identification of pathogenic strains and their differentiation from commensal strains, it is not possible to properly select a therapy - e.g. choice of an antibiotic to which pathogenic strains are sensitive. As APEC strains are characterized by high antibiotic resistance, information on the pathogenicity of the strain is extremely important (Subedi, M. et al., 2018).

Identification of virulence factors may be an important marker for the detection and characterization of APEC strains. The most promising method of their detection is genotyping using multiplex PCR. The usefulness of this method in the identification of APEC is confirmed by numerous reports.

Iguchi et al. developed a multiplex PCR reaction consisting of 20 reaction mixes containing from 6 to 9 primer pairs to identify and classify most of the known *E. coli* O serotypes. Validation of the method using reference and wild-type serotypes showed the accuracy of the method at 100 and 90.8%, respectively (Iguchi, A. et al., 2015).

The multiplex PCR method was also used in the studies of Barbieri et al., who performed the characterization of 144 *E. coli* isolates derived from cellulitis in chickens in southern Brazil. Genotyping based on 34 virulence genes confirmed the presence of genes responsible for adhesion, iron acquisition and serum resistance in all isolates, the presence of which is associated with pathogenicity in poultry (Barbieri, N.L. et al., 2013). In another multiplex PCR method, Ewers et al. used a combination of *papC*, *iucD*, *irp2*, *tsh*, *vat*, *astA*, *iss* and *cva* / *cvi* genes to identify *E. coli* strains isolated from colibacillosis cases. In order to verify the method, the frequency of the studied genes was checked in strains isolated from healthy chickens and in uropathogenic, enteropathogenic and enterotoxic strains. The obtained results confirmed the presence of 4 to 8 tested genes in *E. coli* strains isolated from animals with symptoms of colibacillosis while in non-pathogenic strains the presence of up to 3 studied genes was confirmed (Ewers, C. et al., 2005). Similar observations confirming the higher frequency of virulence genes (5 to 8) in APEC strains were provided by Roussan et al. The same study showed the presence of less than 4 virulence genes in non-pathogenic strains (Roussan, D.A. et al., 2014).

The higher frequency of 2 virulence genes: *iss* (serum resistance gene) and *tsh* (gene encoding heat-sensitive hemagglutinin) in *E. coli* isolates from sick birds was also confirmed by studies in which the *iss*, *tsh*, *iucC* and *cvi* genes were assessed (Skyberg, JA. Et al., 2003).

The work of Westhuizen et al. describes the multiplex PCR method in which a combination of 18 virulence genes was used to perform the molecular characterization of *E. coli* strains in isolates from South Africa and Zimbabwe. The selection of genes was carried out on the basis of the available literature data, which confirmed the high frequency of occurrence of the studied genes among APECs in other countries, and studies on the role of these genes in the virulence mechanism (van der Westhuizen, WA. & Bragg, RR, 2012).

Studies conducted on 219 *E. coli* strains (153 APEC strains, 30 avian fecal *E. coli* (AFEC) and 36 environmental strains) showed a significant advantage of *iroN*, *ompT*, *hlyF*, *iss*, *iutA* virulence genes among APECs (89.5- 94.7%, P <0.001) compared to AFEC (Avian Fecal *Escherichia coli)* (46.653.3%) and environmental strains (10-25%) (Hussei, AH et al., 2013).

Also in the patent literature many studies refer to *E. coli* pathogenic for poultry. The patent (EP2298798B1) describes a method of isolating a nucleic acid containing the *yqi* gene sequence, used in the diagnosis of diseases caused by APEC.

The patent application US 2014/0193824 A1 describes a genetic typing method for *E. coli* based on the modified MLST (Multilocus Sequence Typing) method, which relies on determining the nucleotide sequence of the fimbrial adhesin type 1 gene in the tested sample and further selecting the gene from the group consisting of genes: *fumC*, *adk*, *gyrB*, *icd*, *mdh*, *purA*, and *recA* to identify the *E. coli* clonotype.

Lee et al. developed a method involving the use of DNA chips for detection and identification of *E. coli,* containing sequences specific for these bacteria (WO2008 / 084888 A).

Weigl et al. (US 2008/0199877 A1) developed a method for species specific identification of *Enterobacteriaceae* bacteria (*Escherichia coli*, *Citrobacter freundii*, *Enterobacter aerogenes*, *Enterobacter cloacae*, *Klebsiella oxytoca*, *Klebsiella pneumoniae*, *Providencia stuartii* and *Serratia marcescens*) as well as diagnosis of infections caused by these bacteria and their resistance to quinolones (US 2008/0199877 A1).

In the patent application by Lee et al. a hybridization technique that uses nucleic acid probes complementary to specific regions in the genes encoding rRNA was applied. This method can be used to detect and identify infections that are not caused by viruses (US 2007/0065817 A1).

Detailed APEC identification is a key to understanding the role of virulence factors in the pathogenesis of disease caused by these microorganisms. However, knowledge about these issues is still insufficient to fully understand the relationship between the presence of virulence factors and the ability to induce specific lesions (Janßen, T. et al., 2003). Identification of *E. coli* strains pathogenic for birds is also important due to the possibility of using alternative therapies, e.g. bacteriophages (van der Westhuizen et al., 2012).

Diagnostic tests developed so far do not allow for a quick and unambiguous diagnosis of APEC, taking into account both virulence factors and the most common serotypes, which may make difficult, for example, the implementation of new treatments. The large variety of *E. coli* strains and the fact that some of them are not virulent, are additional factors that cause difficulties to identify these bacteria, considering the division into pathogenic and non-pathogenic strains (Guabiraba, R. & Schouler, C., 2015). Therefore, the problem that still needs to be solved is the unequivocal classification of *E. coli* as APEC, thanks to which it will be possible to increase not only food safety, but also human and animal health.

A particular object of the invention is to provide an efficient APEC identification method which allows the detection of at least 90% and preferably more than 95% of *E. coli* strains pathogenic to poultry. In the context of the present description, a bacterial strain of *E. coli* is considered to be a pathogenic poultry strain that causes a chicken embryo mortality of at least 85% in the 10th day after infection, in a mortality test in which 9-day-old chicken embryos are infected with one infectious dose containing 5×10⁴ CFU of bacteria / embryo.

Surprisingly, the solution to the problems presented is to use the present invention.

The subject of the invention is a method for the identification of an *E. coli* strain pathogenic to birds (APEC), characterized in that:
a) DNA is isolated from the tested sample of biological material,
b) the isolated DNA sample is tested for the presence of: the *iroC* and *hlyF* virulence genes and the gene encoding the 078 serotype,
c) the presence of at least one gene among the mentioned virulence genes or the gene encoding the serotype 078 proves the identification of the APEC strain in the tested sample.

Preferably, in step a) the tested sample is a tissue sample of a sick bird or an environmental sample.

Preferably, in step b) the isolated DNA sample comprises bacterial genomic DNA, especially *E. coli* DNA.

Preferably, in step b) the presence of mentioned genes is verified by PCR.

Preferably, in step b) a multiplex PCR method is used together with the primers set shown as Seq. ID No. 1-6, where:
- the presence of the *iroC* virulence gene is indicated by the presence of a 732 bp product,
- the presence of the *hlyF* virulence gene is indicated by the presence of a 458 bp product, and
- the presence of the antigen gene encoding the 078 serotype is indicated by the presence of a 994 bp product.

Preferably, the identification of the APEC strain means the confirmation of colibacillosis in a sick bird, especially breeding poultry, preferably chickens.

Preferably, non-detection of any of mentioned genes indicates infection with a non-pathogenic strain.

Another object of the invention is a kit for the identification of an *E. coli* strain pathogenic to birds (APEC), characterized in that it contains:
- an oligonucleotide containing at least 20 consecutive nucleotides belonging to the *iroC* virulence gene shown in Seq. ID No. 7,
- an oligonucleotide comprising at least 20 consecutive nucleotides belonging to the *hlyF*virulence gene shown in Seq. ID No. 8,
- an oligonucleotide containing at least 20 consecutive nucleotides encoding the antigen gene of serotype 078 shown in Seq. ID No. 9.

Preferably, the kit of the invention comprises oligonucleotides with the sequences shown as Seq. ID No. 1-6.

Preferably, the kit according to the invention is for the diagnosis of colibacillosis in birds, in particular breeding poultry, preferably chickens.

Another object of the invention is the use of a kit comprising:
- an oligonucleotide containing at least 20 consecutive nucleotides belonging to the *iroC* virulence gene shown in Seq. ID No. 7,
- an oligonucleotide comprising at least 20 consecutive nucleotides belonging to the *hlyF* virulence gene shown in Seq. ID No. 8,
- an oligonucleotide containing at least 20 consecutive nucleotides belonging to the gene of the antigen responsible for serotype 078 shown in Seq. ID No. 9,
for determining the degree of pathogenicity of *E. coli* strain for birds.

Preferably, the mentioned kit contains oligonucleotides with the sequences shown as Seq. ID No. 1-6.

### Detailed description of the invention

A rapid diagnostic method has been revealed. It identifies *E. coli* strains pathogenic for poultry (APEC - Avian Pathogenic *Escherichia coli*) based on detection of a unique combination of two virulence genes and one gene encoding the 078 serotype, selected on the basis of the analysis in which the strains were classified as pathogenic using an non-human embryo mortality of at least 85%, preferably> 96%, on day 19^{th} and reproducibility of the results as criterion. In a favorable implementation of the method it is characterized by the fact that:
a) a bacterial strain of *E. coli* has been isolated from the organs of a sick bird,
b) the isolation of genomic DNA is carried out (any method chosen),
c) a diagnostic test is performed: a multiplex PCR for the presence of two virulence genes (*iroC, hlyF*) and one gene selected from the cluster of genes responsible for the synthesis of the O antigen for serotype O78,
d) the presence of genes in samples is verified during electrophoresis and then the strain is classified into the pathogenicity group: P - pathogenic (presence of at least one of the genes), NP - non-pathogenic (commensal: no gene present).

Any gene belonging to the gene cluster responsible for synthesizing the O antigen for serotype 078 is considered to be the gene responsible for serotype 078.

The unexpectedly disclosed method is capable of easy, quick and unambiguous identification of strains as APEC with an accuracy of up to 97.7%, which is important in the diagnosis of colibacillosis, which is one of the most important causes of poultry losses. It also allows for the selection of appropriate strains, e.g. for the production of an effective autovaccine or the selection of an appropriate therapy. Moreover, equally unexpectedly, the disclosed method is capable of easy and quick identification of *E. coli* strains as non-pathogenic with an accuracy of up to 94.1%.

In order to better explain the invention, it has been illustrated in the examples.

**Example 1.** Characterization of *E. coli* strains based on their sequenced genomes and virulence factors. *Isolation and study of the similarity of strains.*

Initially, a collection of 102 *E. coli* strains isolated from sick birds and 32 *E. coli* strains isolated from healthy birds was obtained. Then, genomic DNA was isolated from each of these strains and the similarity of the strains was tested on the basis of the profiles obtained by MP-PCR (Krawczyk, B. et al., 2006). The strains classified as different form a collection that includes 85 strains obtained from sick birds and 27 strains obtained from healthy birds **(Table 1).**

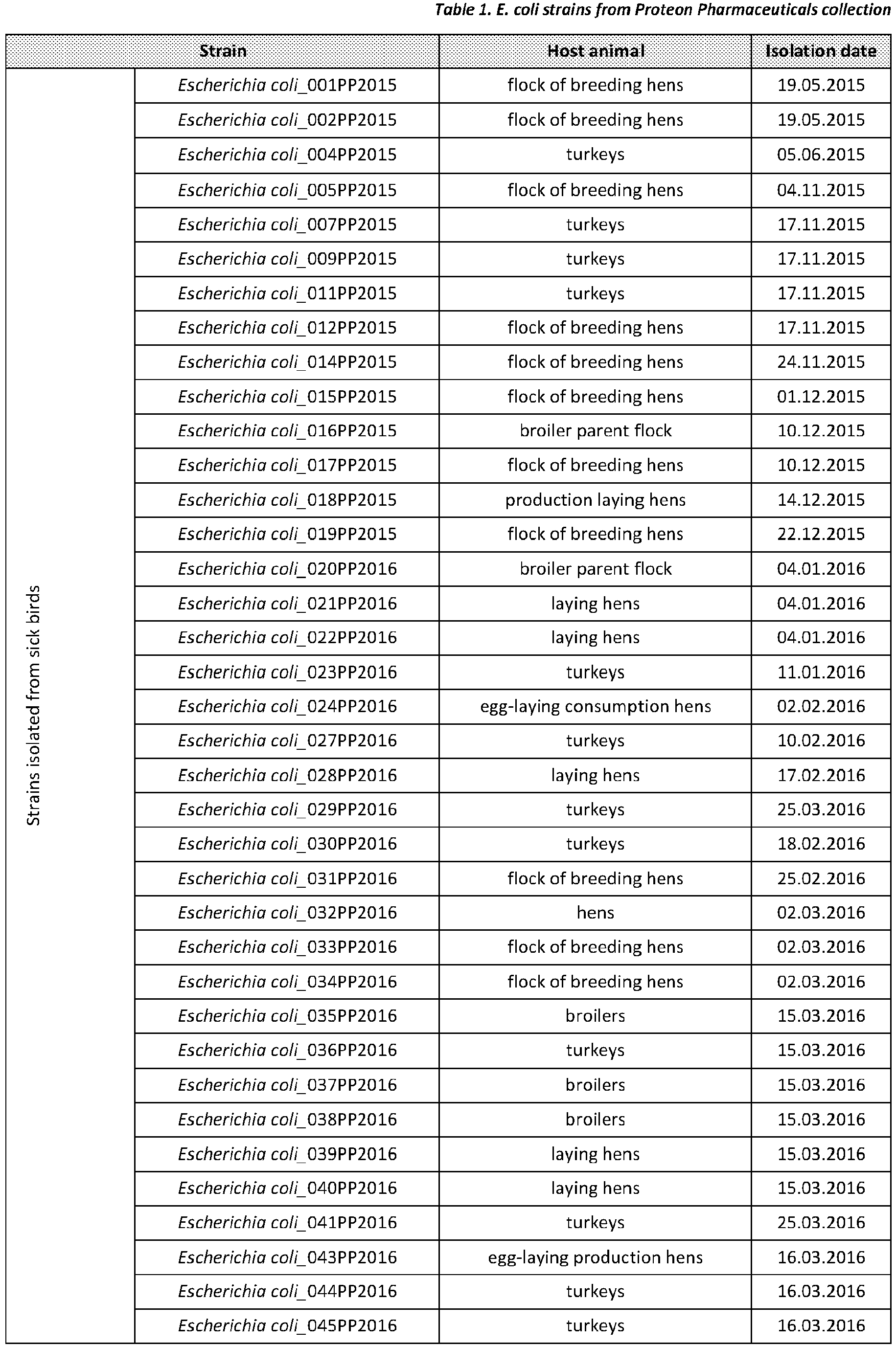

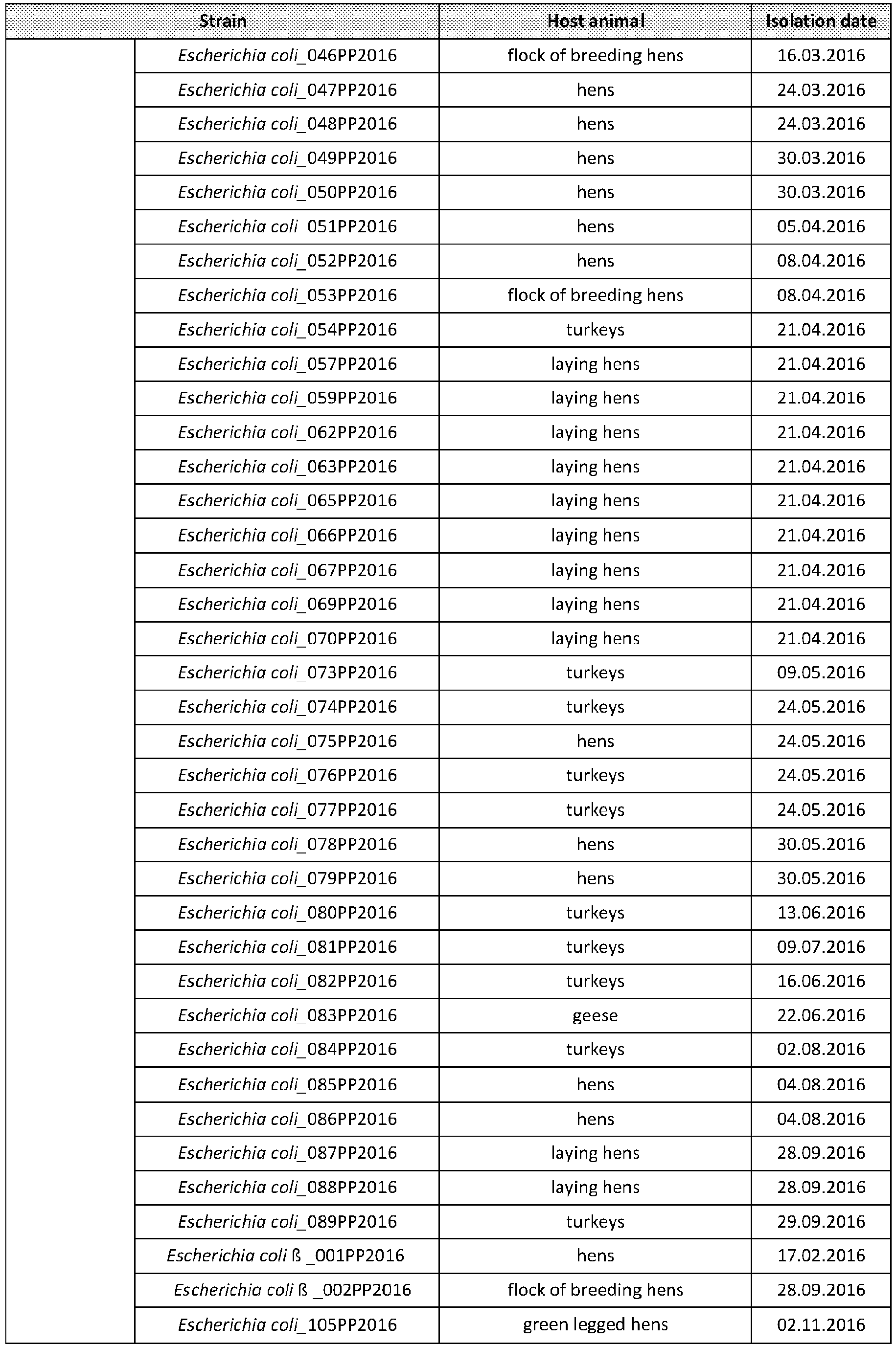

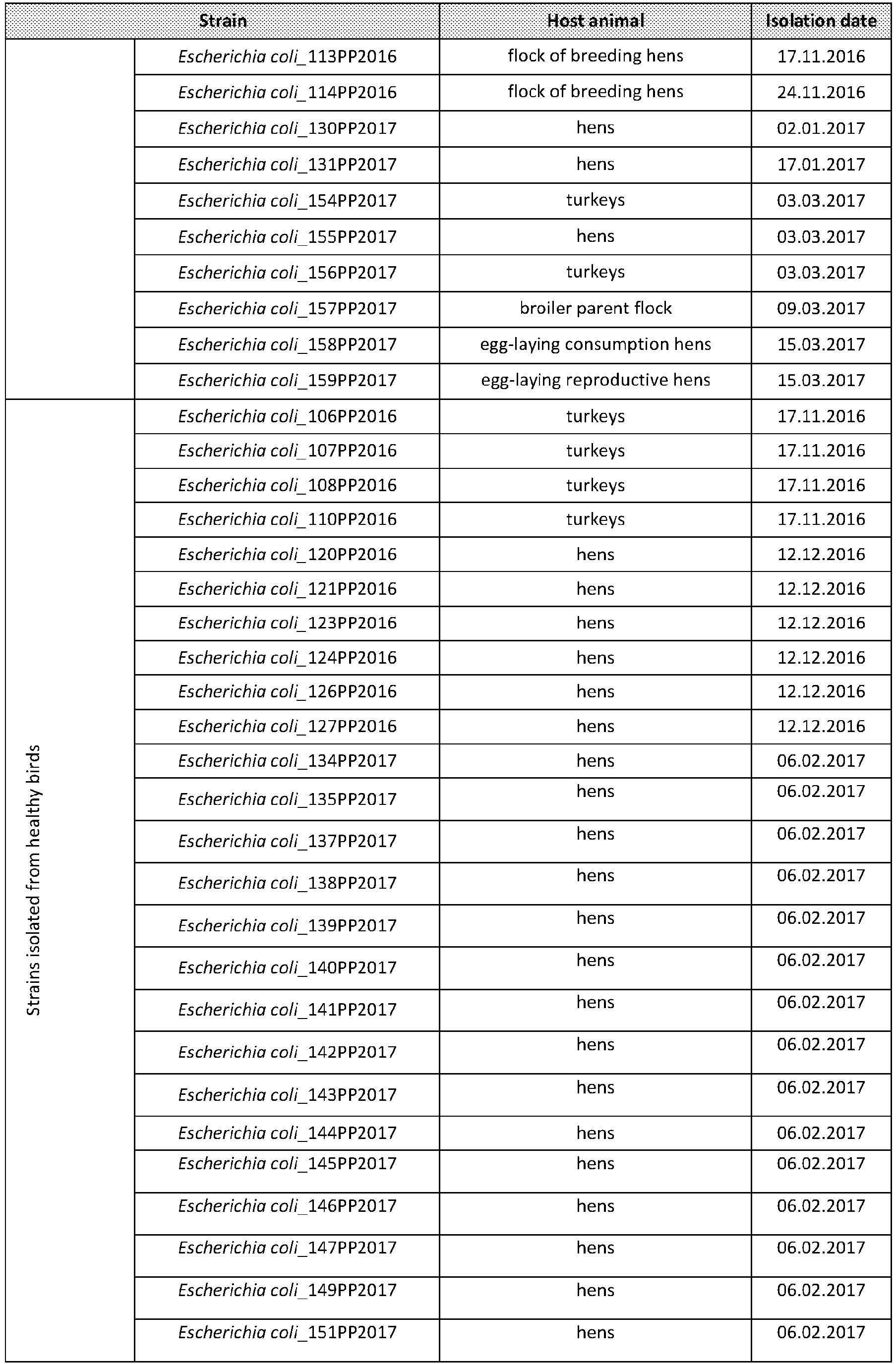

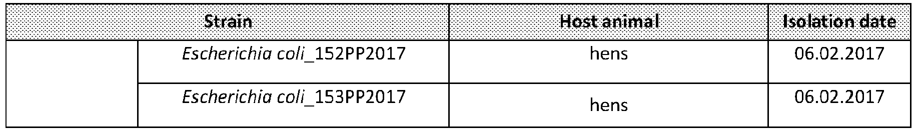

*Strain sequencing and post-sequential processing.*

The DNA of strains was sequenced using the NGS (Next Generation Sequencing) method. The obtained results were *de novo* assembled (SPAdes 3.7.1 and 3.8.0), manually processed (FA_TOOL) and annotated with RAST. Each sequence was saved as a fasta file (both nucleotide and amino acid sequences).

*Analysis of 175 virulence genes in sequenced genomes.*

Based on literature reports, a list of 175 virulence factors found in APEC strains was prepared **(Table 2).** The amino acid sequence for each of the investigated genes was recorded from UniProt database in fasta format.

**Table 2. Virulence factors of APEC strains**

| **Gene group** | **Gene name** |
|---|---|
| adhesin | aufA, aufB, aufC, aufD, aufE, auff, aufG, crI, csgA, csgB, csgC, csgD, csgE, csgF, csgG, eaeH, ecpA, ecpB, ecpC, ecpD, ecpE, ecpR, fimA, fimB, fimC, fimD, fimE, fimF, fimG, fimH, fimI, fmlA, fmlD, focA, focB, focC, focD, focI, hcpA, hcpB, hra, htrA, htrB, htrC, htrE, mat, papA, papB, papC, papD, papE, papF, papGII, papHpapI, papK, papX, sfaB, sfaC, sfaG, sfaH, stgA, stgB, stgD, tsh, yehA, yehB, yehC, yehD, yehE, yqi |
| invasin | ibeA, ibeB, ibeC, ibeR, tia |
| iron-related | chuA, chuS, chuT, chuU, chuW, chuX, chuY, eitA, eitB, eitC, eitD, feoA, feoB, feoC, fepA, fepB, fepC, fepD, fepE, fyuA, ireA, iroB, iroC, iroD, iroE, iroN, irp1, irp2, iucA, iucB, iucC, iucD, iutA, sitA, sitB, site, sitD, ybtA, ybtE, ybtP, ybtS, ybtT, ybtU, ybtX |
| miscellaneous | etsA, etsB, etsC, fliC, malX |
| protectin | bor, kpsE, kpsM, kpsT, neuC, neuS, ompT, traT |
| secretion protein | aec14, aec15, aec16, aec17, aec18, aec19, aec22, aec23, aec24, aec25, aec26, aec27, aec28, aec29, aec30, aec31, aec32, aec7, aec8 |
| toxin | astA, astB, astC, astD, astE, cba, cbi, cdtA, cdtB, cdtC, cma, cmi, cvaA, cvaB, cvaC, hlyD, hlyE, hlyF, pic, pilQ, sat, usp, vat |

Subsequently, based on amino acid sequences, the presence of individual virulence factors in the annotated genomic sequences was assessed. The study allowed for the simultaneous analysis of all selected virulence factors in all analysed genomes. The presence of virulence factors was established as a 0/1 matrix, where 0 stands for lack of a given factor, according to made assumptions, and 1 denotes presence of a given factor, according to made assumptions.

*In silico serotyping based on sequenced genomes.*

In order to associate tested strains to particular serotypes and to check, whether some serotypes are related to the pathogenicity of the strains, *in silico* serotype analysis was performed.

**Example 2.** Classification of *E. coli* strains as pathogenic/non-pathogenic, on the basis of data obtained from *in ovo* embryo viability test.

In order to verify the original assignment of strains to pathogenic and commensal groups (which was based on isolation from either diseased or healthy birds), an *in ovo* test for the viability of the embryos was performed. Experiment 1 aimed at optimizing the infectious dose, while further experiments assessed the pathogenicity of strains.

### Course of experiment 1 (UR, Kraków) - optimization of the infectious dose

The experiment involved infection of 9-day-old chicken embryos with four strains of *E. coli* (2 strains originally classified as non-pathogenic: the reference K-12 strain and 139PP2017, as well as 2 strains originally classified as pathogenic: 002PP2015 and 053PP2016) in 4 infection doses: 1×10⁷ CFU/embryo, 1×10⁶ CFU/embryo, 1×10⁵ CFU/embryo and 1×10⁴ CFU/embryo (60 embryos for each strain and each tested dose).

Embryos were infected with 100 µl/egg of bacterial suspensions, diluted in saline 24h prior to injection.

1080 embryonated eggs were divided into 18 groups of 60 eggs. 16 groups were infected with the tested strains, and 2 additional groups were controls: a null negative control (untreated) and negative control (0.85% NaCl, in which the bacterial suspensions were diluted).

The embryo mortality was assessed in the following days. The experiment was terminated on 10^{th} day after infection.

The embryo mortality data at the end of the experiment are shown in **Table 3.**

The obtained results confirmed the pathogenicity of 2 *E. coli* strains (denoted as 002PP2015 and 053PP2016), for which high embryo mortality was observed, as well as a significantly lower effect of the two remaining strains. Additionally, the obtained data allowed for selection of infectious dose of 5x10°CFU/embryo.

**Table 3. Chicken embryos mortality in experiment 1**

| ***E*. *coli* strain** | **infection dose [CFU/embryo]** | **Mortality 5 days after infection (E14) [%]** | **Mortality at the end of experiment (E19) [%]** | **Standard deviation [%]** |
|---|---|---|---|---|
| K-12 | 1x10⁷ | 43.3 | 79.6 | 12.7 |
| 139PP2017 | | 80.7 | 82.6 | 17.7 |
| 002PP2015 | | 91.7 | 98.3 | 4.1 |
| 053PP2016 | | 98.3 | 100.0 | 0.0 |
| K-12 | 1×10⁶ | 68.7 | 78.9 | 12.2 |
| 139PP2017 | | 48.5 | 64.6 | 28.6 |
| 002PP2015 | | 89.6 | 100.0 | 0.0 |
| 053PP2016 | | 79.1 | 100.0 | 0.0 |
| K-12 | 1×10⁵ | 77.5 | 86.0 | 10.2 |
| 139PP2017 | | 51.3 | 79.6 | 15.2 |
| 002PP2015 | | 91.7 | 100.0 | 0.0 |
| 053PP2016 | | 96.7 | 98.3 | 4.1 |
| K-12 | 1×10⁴ | 63.1 | 72.0 | 4.63 |
| 139PP2017 | | 36.7 | 65.0 | 20.7 |
| 002PP2015 | | 84.6 | 100.0 | 0.0 |
| 053PP2016 | | 96.7 | 100.00 | 0.0 |

### Course of experiment 2 (UR, Kraków)

The experiment involved infection of 9-day chicken embryos with sixteen *E. coli* strains (004PP2015, 009PP2015, 011PP2015, 015PP2015, 039PP2016, 047PP2016, 053PP2016, 075PP2016, 077PP2016, 082PP2016, 087PP2016, 105PP2016, 108PP2016, 138PP2017, 139PP2017 and 144PP2017) at 5×10⁴ CFU/embryo infectious dose (60 embryos for each tested strain, bacterial titer 5x10⁵ CFU/ml).

The embryos were infected with 100 µl/egg of bacterial suspensions, diluted in saline 24h prior to injection. 1080 embryonated eggs were divided into 18 groups, of 60 eggs each. 16 groups were infected with the tested strains, and 2 additional groups were controls: a null negative control (untreated) and negative control (0.85% NaCl, in which the bacterial suspensions were diluted).

The embryo mortality was assessed in the following days. The experiment was terminated on day 10 after infection.

The embryo mortality data at the end of the experiment are shown in **Table 4.**

The obtained results showed that embryo mortality is associated with strain pathogenicity classification. Data in Table 4 were sorted for clarity with descending mortality values. As it can be seen, some strains cause the death of 88.3-100% of the embryos in the selected dose, while other strains at the same dose cause the death of 63.4 - 85% of the embryos, which is a noticeable difference.

**Table 4. Summary of the results of the experiment 2.**

| ***E. coli* strain** | **Mortality 5 days after infection (E14) [%]** | **Mortality at the end of experiment (E19) [%]** | **Standard deviation [%]** |
|---|---|---|---|
| 0 -negative control | 3.5 | 3.5 | 5.5 |
| K-0.85% NaCl | 23.3 | 29.2 | 16.0 |
| 009PP2015 | 100.0 | 100.0 | 0.0 |
| 015PP2015 | 100.0 | 100.0 | 0.0 |
| 039PP2016 | 100.0 | 100.0 | 0.0 |
| 053PP2016 | 100.0 | 100.0 | 0.0 |
| 087PP2016 | 100.0 | 100.0 | 0.0 |
| 004PP2015 | 98.3 | 100.0 | 0.0 |
| 075PP2016 | 98.3 | 100.0 | 0.0 |
| 105PP2016 | 95.0 | 100.0 | 0.0 |
| 144PP2017 | 91.7 | 100.0 | 0.0 |
| 077PP2016 | 88.3 | 95.0 | 8.4 |
| 108PP2016 | 71.7 | 89.8 | 6.3 |
| 047PP2016 | 60.0 | 88.3 | 11.7 |
| 138PP2017 | 60.0 | 85.0 | 8.4 |
| 011PP2015 | 55.0 | 78.3 | 19.4 |
| 139PP2017 | 35.0 | 73.3 | 5.2 |
| 082PP2016 | 21.7 | 63.4 | 13.1 |

### Course of experiment 3 (UR, Kraków)

The experiment involved infection of 9-day chicken embryos with twenty two *E. coli* strains (002PP2015, 017PP2015, 018PP2015, 022PP2016, 029PP2016, 030PP2016, 032PP2016, 036PP2016, 040PP2016, 044PP2016, 045PP2016, 048PP2016, 049PP2016, 051PP2016, 054PP2016, 063PP2016, 070PP2016, 074PP2016, 076PP2016, 084PP2016, 110PP2016 and 120PP2016) at 5x10⁴ CFU/embryo infectious dose (30 embryos for each tested strain, bacterial titer 5x10⁵ CFU/ml).

The embryos were infected with 100 µl/egg of bacterial suspensions, diluted in saline 24h prior to injection. 720 embryonated eggs were divided into 24 groups, of 30 eggs each. 22 groups were infected with the tested strains, and 2 additional groups were controls: a null negative control (untreated) and negative control (0.85% NaCl, in which the bacterial suspensions were diluted).

The embryo mortality was assessed in the following days. The experiment was terminated on day 10 after infection.

The embryo mortality data at the end of the experiment are shown in **Table 5.**

The obtained results showed that embryo mortality is associated with strain pathogenicity classification. Data in Table 5 were sorted for clarity with descending mortality values. As it can be seen, some strains cause the death of 93.3-100% of the embryos in the selected dose, while other strains at the same dose cause the death of 52.2-83.3% of the embryos, which is a noticeable difference.

**Table 5. Summary of the results of the experiment 3.**

| ***E. coli* strain** | **Mortality 5 days after infection (E14) [%]** | **Mortality at the end of experiment (E19) [%]** | **Standard deviation [%]** |
|---|---|---|---|
| 0 - negative control | 0.0 | 0.0 | 0.0 |
| K - 0.85% NaCl | 13.3 | 13.3 | 15.3 |
| 002PP2015 | 100.0 | 100.0 | 0.0 |
| 017PP2015 | 100.0 | 100.0 | 0.0 |
| 022PP2016 | 100.0 | 100.0 | 0.0 |
| 040PP2016 | 100.0 | 100.0 | 0.0 |
| 044PP2016 | 100.0 | 100.0 | 0.0 |
| 076PP2016 | 100.0 | 100.0 | 0.0 |
| 029PP2016 | 96.7 | 100.0 | 0.0 |
| 045PP2016 | 96.7 | 100.0 | 0.0 |
| 070PP2016 | 95.8 | 100.0 | 0.0 |
| 063PP2016 | 92.6 | 100.0 | 0.0 |
| 018PP2015 | 91.1 | 100.0 | 0.0 |
| 110PP2016 | 83.3 | 100.0 | 0.0 |
| 030PP2016 | 82.6 | 100.0 | 0.0 |
| 084PP2016 | 80.0 | 100.0 | 0.0 |
| 054PP2016 | 86.7 | 96.7 | 5.8 |
| 036PP2016 | 86.3 | 96.7 | 5.8 |
| 032PP2016 | 61.1 | 93.3 | 11.6 |
| 074PP2016 | 72.6 | 83.3 | 20.8 |
| 051PP2016 | 62.6 | 75.9 | 5.3 |
| 049PP2016 | 47.8 | 74.8 | 28.1 |
| 048PP2016 | 36.3 | 57.4 | 17.9 |
| 120PP2016 | 27.4 | 52.2 | 13.5 |

### Course of experiment 4 (UR, Kraków)

The experiment involved infection of 9-day chicken embryos with twenty two *E. coli* strains (014PP2015, 024PP2016, 035PP2016, 043PP2016, 050PP2016, 057PP2016, 078PP2016, 079PP2016, 080PP2016, 081PP2016, 086PP2016, 114PP2016, 135PP2017, 137PP2017, 141PP2017, 142PP2017, 143PP2017, 151PP2017, 152PP2017, 155PP2017, 159PP2017 and β001PP2016) at 5x10⁴ CFU/embryo infectious dose (30 embryos for each tested strain, bacterial titer 5×10⁵ CFU/ml).

Z The embryos infected with 100 µl/egg of bacterial suspensions, diluted in saline 24h priorto injection. 720 embryonated eggs were divided into 24 groups, of 30 eggs each. 22 groups were infected with the tested strains, and 2 additional groups were controls: a null negative control (untreated) and negative control (0.85% NaCl, in which the bacterial suspensions were diluted).

The embryo mortality was assessed in the following days. The experiment was terminated on day 10 after infection.

The embryo mortality data at the end of the experiment are shown in **Table 6.**

The obtained results showed that embryo mortality is associated with strain pathogenicity classification. Data in Table 6 were for clarity sorted with descending mortality values. As it can be seen, some strains cause the death of 93.3-100% of the embryos in the selected dose, while other strains at the same dose cause the death of 70-83.3% of the embryos, which is a noticeable difference.

**Table 6. Summary of the results of the experiment 4.**

| ***E. coli* strain** | **Mortality 5 days after infection (E14) [%]** | **Mortality at the end of experiment (E19) [%]** | **Standard deviation [%]** |
|---|---|---|---|
| 0 - negative control | 0.0 | 0.0 | 0.0 |
| K-0.85% NaCl | 3.4 | 3.7 | 6.4 |
| 014PP2015 | 100.0 | 100.0 | 0.0 |
| 024PP2016 | 100.0 | 100.0 | 0.0 |
| 035PP2016 | 100.0 | 100.0 | 0.0 |
| 050PP2016 | 100.0 | 100.0 | 0.0 |
| 086PP2016 | 100.0 | 100.0 | 0.0 |
| 159PP2017 | 100.0 | 100.0 | 0.0 |
| B001PP2016 | 100.0 | 100.0 | 0.0 |
| 043PP2016 | 90.0 | 100.0 | 0.0 |
| 078PP2016 | 96.7 | 100.0 | 0.0 |
| 079PP2016 | 96.7 | 100.0 | 0.0 |
| 080PP2016 | 96.7 | 100.0 | 0.0 |
| 155PP2016 | 93.4 | 100.0 | 0.0 |
| 081PP2016 | 86.7 | 100.0 | 0.0 |
| 152PP2017 | 80.0 | 100.0 | 0.0 |
| 057PP2016 | 96.3 | 96.3 | 6.4 |
| 137PP2017 | 93.4 | 96.3 | 6.4 |
| 151PP2017 | 93.4 | 96.3 | 6.4 |
| 143PP2017 | 80.0 | 96.3 | 6.4 |
| 114PP2016 | 90.0 | 93.3 | 11.5 |
| 142PP2017 | 76.7 | 93.3 | 11.5 |
| 135PP2017 | 56.7 | 83.3 | 5.8 |
| 141PP2017 | 69.0 | 70.0 | 26.5 |

### Course of experiment 5 (UR, Kraków)

The experiment involved infection of 9-day chicken embryos with twenty eight *E. coli* strains (001PP2015, 005PP2015, 007PP2015, 027PP2016, 052PP2016, 059PP2016, 069PP2016, 083PP2016, 085PP2016, 106PP2016, 107PP2016, 113PP2016, 123PP2016, 124PP2016, 126PP2016, 127PP2016, 130PP2017, 131PP2017, 134PP2017, 140PP2017, 145PP2017, 146PP2017, 147PP2017, 149PP2017, 153PP2017, 154PP2017, 156PP2017 and B002PP2016) at 5x10⁴ CFU/embryo infectious dose (30 embryos for each tested strain, bacterial titer 5x10⁵ CFU/ml).

The embryos were infected with 100 µl/egg of bacterial suspensions, diluted in saline 24h prior to injection. 900 embryonated eggs were divided into 30 groups, of 30 eggs each. 28 groups were infected with the tested strains, and 2 additional groups were controls: a null negative control (untreated) and negative control (0.85% NaCl, in which the bacterial suspensions were diluted).

The embryo mortality was assessed in the following days. The experiment was terminated on day 10 after infection.

The embryo mortality data at the end of the experiment are shown in **Table 7.**

The obtained results showed that embryo mortality is associated with strain pathogenicity classification. Data in Table 7 were for clarity sorted with descending mortality values. As it can be seen, some strains cause the death of 93.3-100% of the embryos in the selected dose, while other strains at the same dose cause the death of 34.4-83.3% of the embryos, which is a noticeable difference.

**Table 7. Summary of the results of the experiment 5.**

| ***E. coli* strain** | **Mortality 5 days after infection (E14) [%]** | **Mortality at the end of experiment (E19) [%]** | **Standard deviation [%]** |
|---|---|---|---|
| 0 - negative control | 0.0 | 0.0 | 0.0 |
| K-0.85% NaCl | 16.7 | 16.7 | 15.3 |
| 154PP2017 | 100.0 | 100.0 | 0.0 |
| 156PP2017 | 100.0 | 100.0 | 0.0 |
| 005PP2015 | 95.8 | 100.0 | 0.0 |
| 123PP2016 | 93.3 | 100.0 | 0.0 |
| 124PP2016 | 93.3 | 100.0 | 0.0 |
| 146PP2017 | 93.3 | 100.0 | 0.0 |
| 145PP2017 | 92.6 | 100.0 | 0.0 |
| 126PP2016 | 86.7 | 100.0 | 0.0 |
| 106PP2016 | 83.3 | 100.0 | 0.0 |
| 131PP2017 | 82.2 | 100.0 | 0.0 |
| 127PP2016 | 80.0 | 100.0 | 0.0 |
| 134PP2017 | 78.5 | 100.0 | 0.0 |
| 113PP2016 | 76.7 | 100.0 | 0.0 |
| 059PP2016 | 66.7 | 100.0 | 0.0 |
| 069PP2016 | 66.3 | 100.0 | 0.0 |
| 153PP2017 | 65.0 | 100.0 | 0.0 |
| 083PP2016 | 93.3 | 96.7 | 5.8 |
| 085PP2016 | 89.6 | 96.7 | 5.8 |
| 149PP2017 | 90.0 | 93.3 | 5.8 |
| 130PP2017 | 73.3 | 93.3 | 5.8 |
| 107PP2016 | 63.0 | 93.3 | 11.5 |
| B002PP2016 | 53.3 | 93.3 | 5.8 |
| 001PP2015 | 76.7 | 83.3 | 11.5 |
| 052PP2016 | 52.6 | 80.0 | 17.3 |
| 007PP2015 | 54.2 | 70.8 | 8.8 |
| 027PP2016 | 60.0 | 70.0 | 10.0 |
| 147PP2017 | 50.0 | 66.7 | 15.3 |
| 140PP2017 | 16.7 | 34.4 | 15.0 |

### Course of experiment 6 (UR, Kraków)

The experiment involved infection of 9-day chicken embryos with thirty three *E. coli* strains (001PP2015, 012PP2015, 014PP2015, 016PP2015, 019PP2015, 020PP2015, 021PP2016, 023PP2016, 028PP2016, 031PP2016, 032PP2016, 033PP2016, 034PP2016, 038PP2016, 041PP2016, 046PP2016, 047PP2016, 051PP2016, 052PP2016, 062PP2016, 065PP2016, 066PP2016, 067PP2016, 073PP2016, 088PP2016, 089PP2016, 121PP2016, 135PP2017, 137PP2017, 138PP2017, 141PP2017, 157PP2017 and 158PP2017) at 5x10⁴ CFU/embryo infectious dose (30 embryos for each tested strain, bacterial titer 5x10⁵ CFU/ml).

The embryos were infected with 100 µl/egg of bacterial suspensions, diluted in saline 24h prior to injection. 1050 embryonated eggs were divided into 35 groups, of 30 eggs each. 33 groups were infected with the tested strains, and 2 additional groups were controls: a null negative control (untreated) and negative control (0.85% NaCl, in which the bacterial suspensions were diluted).

The embryo mortality was assessed in the following days. The experiment was terminated on day 10 after infection.

The embryo mortality data at the end of the experiment are shown in **Table 8.**

The obtained results showed that embryo mortality is associated with strain pathogenicity classification. Data in Table 8 were for clarity sorted with descending mortality values. As it can be seen, some strains cause the death of 86.7-100% of the embryos in the selected dose, while other strains at the same dose cause the death of 26.7-71.9% of the embryos, which is a noticeable difference.

**Table 8. Summary of the results of the experiment 6.**

| ***E. coli* strain** | **Mortality 5 days after infection (E14) [%]** | **Mortality at the end of experiment (E19) [%]** | **Standard deviation [%]** |
|---|---|---|---|
| 0 - negative control | 0.0 | 10.7 | 0.64 |
| K-0.85% NaCl | 10.0 | 24.1 | 15.1 |
| 016PP2015 | 100.0 | 100.0 | 0.0 |
| 028PP2016 | 100.0 | 100.0 | 0.0 |
| 031PP2016 | 100.0 | 100.0 | 0.0 |
| 033PP2016 | 100.0 | 100.0 | 0.0 |
| 041PP2016 | 100.0 | 100.0 | 0.0 |
| 066PP2016 | 100.0 | 100.0 | 0.0 |
| 067PP2016 | 100.0 | 100.0 | 0.0 |
| 121PP2016 | 100.0 | 100.0 | 0.0 |
| 157PP2017 | 100.0 | 100.0 | 0.0 |
| 046PP2016 | 96.7 | 100.0 | 0.0 |
| 062PP2016 | 96.7 | 100.0 | 0.0 |
| 088PP2016 | 96.7 | 100.0 | 0.0 |
| 158PP2017 | 96.7 | 100.0 | 0.0 |
| 052PP2016 | 86.7 | 100.0 | 0.0 |
| 073PP2016 | 96.7 | 96.7 | 5.8 |
| 135PP2017 | 90.0 | 96.3 | 6.4 |
| 021PP2016 | 86.7 | 96.3 | 6.4 |
| 051PP2016 | 66.7 | 90.0 | 10.0 |
| 012PP2015 | 60.0 | 86.7 | 5.8 |
| 023PP2016 | 60.0 | 71.9 | 17.3 |
| 014PP2015 | 71.3 | 71.3 | 19.7 |
| 089PP2016 | 50.0 | 63.3 | 5.8 |
| 034PP2016 | 40.0 | 56.7 | 15.3 |
| 019PP2015 | 40.0 | 43.3 | 20.8 |
| 038PP2016 | 23.3 | 43.3 | 20.8 |
| 020PP2015 | 43.3 | 43.3 | 23.4 |
| 032PP2016 | 16.7 | 46.3 | 11.6 |
| 138PP2017 | 44.8 | 44.8 | 29.3 |
| 047PP2016 | 13.3 | 42.2 | 29.1 |
| 065PP2016 | 20.0 | 37.5 | 22.2 |
| 137PP2017 | 26.7 | 34.8 | 13.0 |
| 001PP2015 | 20.0 | 31.1 | 20.1 |
| 141PP2017 | 23.3 | 26.7 | 5.8 |

### Course of experiment 7 (UR, Kraków)

The experiment involved infection of 9-day chicken embryos with thirty three *E. coli* strains (011PP2015, 012PP2015, 014PP2015, 019PP2015, 020PP2016, 023PP2016, 027PP2016, 034PP2016, 038PP2016, 049PP2016, 050PP2016, 052PP2016, 059PP2016, 073PP2016, 074PP2016, 075PP2016, 077PP2016, 081PP2016, 084PP2016, 089PP2016, 106PP2016, 108PP2016, 114PP2016, 121PP2016, 123PP2016, 124PP2016, 126PP2016, 127PP2016, 137PP2017, 141PP2017, 146PP2017, 149PP2017 and 154PP2017) at 5x10⁴ CFU/embryo infectious dose (30 embryos for each tested strain, bacterial titer 5x10⁵ CFU/ml).

The embryos were infected with 100 µl/egg of bacterial suspensions, diluted in saline 24h prior to injection. 1050 embryonated eggs were divided into 35 groups, of 30 eggs each. 33 groups were infected with the tested strains, and 2 additional groups were controls: a null negative control (untreated) and negative control (0.85% NaCl, in which the bacterial suspensions were diluted).

The embryo mortality was assessed in the following days. The experiment was terminated on day 10 after infection_

The embryo mortality data at the end of the experiment are shown in **Table 9.**

The obtained results showed that embryo mortality is associated with strain pathogenicity classification. Data in Table 9 were for clarity sorted with descending mortality values. As it can be seen, some strains cause the death of 85.5-100% of the embryos in the selected dose, while other strains at the same dose cause the death of 3.3-82.5% of the embryos, which is a noticeable difference.

**Table 9. Summary of the results of the experiment 7.**

| ***E. coli* strain** | **Mortality 5 days after infection (E14) [%]** | **Mortality at the end of experiment (E19) [%]** | **Standard deviation [%]** |
|---|---|---|---|
| 0 - negative control | 0.0 | 0.0 | 0.0 |
| K - 0.85% NaCl | 0.0 | 3.3 | 5.8 |
| 089PP2016 | 80.0 | 100.0 | 0.0 |
| 074PP2016 | 74.4 | 100.0 | 0.0 |
| 126PP2016 | 71.7 | 100.0 | 0.0 |
| 127PP2016 | 66.7 | 100.0 | 0.0 |
| 124PP2016 | 65.6 | 100.0 | 0.0 |
| 081PP2016 | 42.2 | 100.0 | 0.0 |
| 154PP2017 | 90.0 | 96.7 | 5.8 |
| 038PP2016 | 86.3 | 96.7 | 5.8 |
| 084PP2016 | 85.9 | 96.7 | 5.77 |
| 020PP2016 | 73.3 | 93.3 | 11.5 |
| 023PP2016 | 63.3 | 93.3 | 11.5 |
| 123PP2016 | 60.7 | 93.0 | 6.1 |
| 034PP2016 | 48.1 | 93.0 | 6.1 |
| 059PP2016 | 37.8 | 93.0 | 6.1 |
| 077PP2016 | 75.2 | 92.6 | 12.8 |
| 014PP2015 | 73.1 | 92.6 | 12.8 |
| 012PP2015 | 57.4 | 89.3 | 11.1 |
| 075PP2016 | 60.0 | 88.4 | 11.1 |
| 146PP2017 | 57.8 | 86.3 | 15.2 |
| 027PP2016 | 52.7 | 85.5 | 4.8 |
| 121PP2016 | 51.1 | 82.5 | 10.9 |
| 149PP2017 | 40.0 | 76.7 | 15.3 |
| 019PP2015 | 37.8 | 75.9 | 25.1 |
| 052PP2016 | 63.8 | 73.3 | 37.9 |
| 050PP2016 | 63.3 | 73.3 | 11.5 |
| 011PP2015 | 48.5 | 65.6 | 15.0 |
| 073PP2016 | 38.1 | 58.5 | 20.2 |
| 141PP2017 | 14.9 | 49.0 | 11.9 |
| 137PP2017 | 20.0 | 30.0 | 20.0 |
| 049PP2016 | 13.7 | 20.4 | 9.4 |
| 106PP2016 | 3.3 | 19.9 | 8.7 |
| 108PP2016 | 0.0 | 3.3 | 5.8 |
| 114PP2016 | 3.3 | 3.3 | 5.8 |

### Course of experiment 8 (UR, Kraków)

The experiment involved infection of 9-day chicken embryos with twenty two *E. coli* strains (001PP2015, 019PP2015, 020PP2016, 023PP2016, 027PP2016, 032PP2016, 034PP2016, 037PP2016 - replicate A, 037PP2016 - replicate B, 038PP2016, 047PP2016, 049PP2016, 050PP2016, 051PP2016, 073PP2016, 089PP2016, 106PP2016, 108PP2016, 114PP2016, 138PP2017, 142PP2017 and 149PP2017) at 5x10⁴ CFU/embryo infectious dose (30 embryos for each tested strain, bacterial titre 5x10⁵ CFU/ml).

The embryos were infected with 100 µl/egg of bacterial suspensions, diluted in saline 24h priorto injection. 720 embryonated eggs were divided into 24 groups, of 30 eggs each. 22 groups were infected with the tested strains, and 2 additional groups were controls: a null negative control (untreated) and negative control (0.85% NaCl, in which the bacterial suspensions were diluted).

The embryo mortality was assessed in the following days. The experiment was terminated on day 10 after infection.

The embryo mortality data at the end of the experiment are shown in **Table 10.**

The obtained results showed that embryo mortality is associated with strain pathogenicity classification. Data in Table 10 were for clarity sorted with descending mortality values. As it can be seen, some strains cause the death of 85.6-100% of the embryos in the selected dose, while other strains at the same dose cause the death of 65.7-83% of the embryos, which is a noticeable difference.

**Table 10. Summary of the results of the experiment 8.**

| ***E. coli strain*** | **Mortality 5 days after infection (E14) [%]** | **Mortality at the end of experiment (E19) [%]** | **Standard deviation [%]** |
|---|---|---|---|
| 0 - negative control | no data | 0.0 | 0.0 |
| K - 0.85% NaCl | no data | 7.0 | 6.12 |
| 019PP2015 | no data | 100.0 | 0.0 |
| 023PP2016 | no data | 100.0 | 0.0 |
| 037PP2016 - A | no data | 100.0 | 0.0 |
| 073PP2016 | no data | 100.0 | 0.0 |
| 114PP2016 | no data | 100.0 | 0.0 |
| 149PP2017 | no data | 100.0 | 0.0 |
| 138PP2017 | no data | 100.0 | 0.0 |
| 020PP2016 | no data | 96.7 | 5.8 |
| 037PP2016 - B | no data | 96.7 | 5.8 |
| 038PP2016 | no data | 96.7 | 5.8 |
| 089PP2016 | no data | 96.7 | 5.8 |
| 034PP2016 | no data | 96.3 | 6.4 |
| 106PP2016 | no data | 93.3 | 5.8 |
| 050PP2016 | no data | 93.0 | 6.1 |
| 001PP2015 | no data | 90.0 | 17.3 |
| 108PP2016 | no data | 90.0 | 17.3 |
| 142PP2017 | no data | 90.0 | 10.0 |
| 051PP2016 | no data | 85.6 | 17.1 |
| 027PP2016 | no data | 83.0 | 11.2 |
| 047PP2016 | no data | 83.0 | 5.1 |
| 049PP2016 | no data | 76.9 | 11.2 |
| 032PP2016 | no data | 65.7 | 28.9 |

The original pathogenic classification of the strains basing on the health status of the birds, from which the strain was isolated (bird healthy/diseased), was verified according to the results of the chicken embryo viability test in *in ovo* model. The collection of strains was reclassified based on those results.

After reclassification, 105 strains were selected for the final group for analysis.

**Table 11. Pathogenicity of reclassified strains.**

| ***E. coli* strain** | **Mortality at 5th day after challenge [%]** | **Mortality at the end of experiment [%]** | **SD [%]** | **No. experiment** | **Original classification** | **Classification after *in ovo* test** | **Final classification** |
|---|---|---|---|---|---|---|---|
| **FINAL GROUP OF STRAINS** | | | | | | | |
| 001PP2015 | 76.7 | 83.3 | 11.5 | 5 | P | NP | NP |
| | 20.0 | 31.1 | 20.1 | 6 | | NP | |
| | bd | 90.0 | 17.3 | 8 | | P | |
| 002PP2015 | 91.7 | 98.3 | 4.1 | 1 | P | P | P |
| | 89.6 | 100.0 | 0.0 | 1 | | P | |
| | 91.7 | 100.0 | 0.0 | 1 | | P | |
| | 84.6 | 100.0 | 0.0 | 1 | | P | |
| | 100.0 | 100.0 | 0.0 | 3 | | P | |
| 004PP2015 | 98.3 | 100.0 | 0.0 | 2 | P | P | P |
| 005PP2015 | 95.8 | 100.0 | 0.0 | 5 | P | P | P |
| 007PP2015 | 54.2 | 70.8 | 8.8 | 5 | P | NP | NP |
| 009PP2015 | 100.0 | 100.0 | 0.0 | 2 | P | P | P |
| 011PP2015 | 55.0 | 78.3 | 19.4 | 2 | P | NP | NP |
| | 48.5 | 65.6 | 15.0 | 7 | | NP | |
| 012PP2015 | 60.0 | 86.7 | 5.8 | 6 | P | P | P |
| | 57.4 | 89.3 | 11.1 | 7 | | P | |
| 014PP2015 | 100.0 | 100.0 | 0.0 | 4 | P | P | P |
| | bd | 71.3 | 19.7 | 6 | | NP | |
| | 73.1 | 92.6 | 12.8 | 7 | | P | |
| 015PP2015 | 100.0 | 100.0 | 0.0 | 2 | P | P | P |
| 016PP2015 | 100.0 | 100.0 | 0.0 | 6 | P | P | P |
| 017PP2015 | 100.0 | 100.0 | 0.0 | 3 | P | P | P |
| 018PP2015 | 91.1 | 100.0 | 0.0 | 3 | P | P | P |
| 020PP2015 | bd | 42.2 | 23.4 | 6 | P | NP | P |
| | 73.3 | 93.3 | 11.5 | 7 | | P | |
| | bd | 96.7 | 5.8 | 8 | | P | |
| 021PP2016 | 86.7 | 96.3 | 6.4 | 6 | P | P | P |
| 022PP2016 | 100.0 | 100.0 | 0.0 | 3 | P | P | P |
| 023PP2016 | 60.0 | 71.9 | 17.3 | 6 | P | NP | P |
| | 63.3 | 93.3 | 11.5 | 7 | | P | |
| | bd | 100.0 | 0.0 | 8 | | P | |
| 024PP2016 | 100.0 | 100.0 | 0.0 | 4 | P | P | P |
| 028PP2016 | 100.0 | 100.0 | 0.0 | 6 | P | P | P |
| 029PP2016 | 96.7 | 100.0 | 0.0 | 3 | P | P | P |
| 030PP2016 | 82.6 | 100.0 | 0.0 | 3 | P | P | P |
| 031PP2016 | 100.0 | 100.0 | 0.0 | 6 | P | P | P |
| 032PP2016 | 61.1 | 93.3 | 11.6 | 3 | P | P | NP |
| | 16.7 | 46.3 | 11.6 | 6 | | NP | |
| | bd | 65.7 | 28.9 | 8 | | NP | |
| 033PP2016 | 100.0 | 100.0 | 0.0 | 6 | P | P | P |
| 034PP2016 | 40.0 | 56.7 | 15.3 | 6 | P | NP | P |
| | 48.1 | 93.0 | 6.1 | 7 | | P | |
| | bd | 96.3 | 6.4 | 8 | | P | |
| 035PP2016 | 100.0 | 100.0 | 0.0 | 4 | P | P | P |
| 036PP2016 | 86.3 | 96.7 | 5.8 | 3 | P | P | P |
| 037PP2016 | bd | 100.0 | 0.0 | 8 | P | P | P |
| | bd | 96.7 | 5.8 | 8 | | P | |
| 038PP2016 | 23.3 | 43.3 | 20.8 | 6 | P | NP | P |
| | 86.3 | 96.7 | 5.8 | 7 | | P | |
| | bd | 96.7 | 5.8 | 8 | | P | |
| 039PP2016 | 100.0 | 100.0 | 0.0 | 2 | P | P | P |
| 040PP2016 | 100.0 | 100.0 | 0.0 | 3 | P | P | P |
| 041PP2016 | 100.0 | 100.0 | 0.0 | 6 | P | P | P |
| 043PP2016 | 90.0 | 100.0 | 0.0 | 4 | P | P | P |
| 044PP2016 | 100.0 | 100.0 | 0.0 | 3 | P | P | P |
| 045PP2016 | 96.7 | 100.0 | 0.0 | 3 | P | P | P |
| 046PP2016 | 96.7 | 100.0 | 0.0 | 6 | P | P | P |
| 047PP2016 | 60.0 | 88.3 | 11.7 | 2 | P | P | NP |
| | 13.3 | 42.2 | 29.1 | 6 | | NP | |
| | bd | 83.0 | 5.1 | 8 | | NP | |
| 048PP2016 | 36.3 | 57.4 | 17.9 | 3 | P | NP | NP |
| 049PP2016 | 47.8 | 74.8 | 28.1 | 3 | P | NP | NP |
| | 13.7 | 20.4 | 9.4 | 7 | | NP | |
| | bd | 76.9 | 11.2 | 8 | | NP | |
| 050PP2016 | 100.0 | 100.0 | 0.0 | 4 | P | P | P |
| | 63.3 | 73.3 | 11.5 | 7 | | NP | |
| | bd | 93.0 | 6.1 | 8 | | P | |
| 052PP2016 | 52.6 | 80.0 | 17.3 | 5 | P | NP | NP |
| | 86.7 | 100.0 | 0.0 | 6 | | P | |
| | 63.8 | 73.3 | 37.9 | 7 | | NP | |
| 053PP2016 | 98.3 | 100.0 | 0.0 | 1 | P | P | P |
| | 79.1 | 100.0 | 0.0 | 1 | | P | |
| | 96.7 | 98.3 | 4.1 | 1 | | P | |
| | 96.7 | 100.00 | 0.0 | 1 | | P | |
| | 100.0 | 100.0 | 0.0 | 2 | | P | |
| 054PP2016 | 86.7 | 96.7 | 5.8 | 3 | P | P | P |
| 057PP2016 | 96.3 | 96.3 | 6.4 | 4 | P | P | P |
| 059PP2016 | 66.7 | 100.0 | 0.0 | 5 | P | P | P |
| | 37.8 | 93.0 | 6.1 | 7 | | P | |
| 062PP2016 | 96.7 | 100.0 | 0.0 | 6 | P | P | P |
| 063PP2016 | 92.6 | 100.0 | 0.0 | 3 | P | P | P |
| 065PP2016 | 20.0 | 37.5 | 22.2 | 6 | P | NP | NP |
| 066PP2016 | 100.0 | 100.0 | 0.0 | 6 | P | P | P |
| 067PP2016 | 100.0 | 100.0 | 0.0 | 6 | P | P | P |
| 069PP2016 | 66.3 | 100.0 | 0.0 | 5 | P | P | P |
| 070PP2016 | 95.8 | 100.0 | 0.0 | 3 | P | P | P |
| 073PP2016 | 96.7 | 96.7 | 5.8 | 6 | P | P | P |
| | 38.1 | 58.5 | 20.2 | 7 | | NP | |
| | bd | 100.0 | 0.0 | 8 | | P | |
| 075PP2016 | 98.3 | 100.0 | 0.0 | 2 | P | P | P |
| | 60.0 | 88.4 | 11.1 | 7 | | P | |
| 076PP2016 | 100.0 | 100.0 | 0.0 | 3 | P | P | P |
| 077PP2016 | 88.3 | 95.0 | 8.4 | 2 | P | P | P |
| | 75.2 | 92.6 | 12.8 | 7 | | P | |
| 078PP2016 | 96.7 | 100.0 | 0.0 | 4 | P | P | P |
| 079PP2016 | 96.7 | 100.0 | 0.0 | 4 | P | P | P |
| 080PP2016 | 96.7 | 100.0 | 0.0 | 4 | P | P | P |
| 081PP2016 | 86.7 | 100.0 | 0.0 | 4 | P | P | P |
| | 42.2 | 100.0 | 0.0 | 7 | | P | P |
| 082PP2016 | 21.7 | 63.4 | 13.1 | 2 | P | NP | NP |
| 083PP2016 | 93.3 | 96.7 | 5.8 | 5 | P | P | P |
| 084PP2016 | 80.0 | 100.0 | 0.0 | 3 | P | P | P |
| | 85.9 | 96.7 | 5.77 | 7 | | P | |
| 085PP2016 | 89.6 | 96.7 | 5.8 | 5 | P | P | P |
| 086PP2016 | 100.0 | 100.0 | 0.0 | 4 | P | P | P |
| 087PP2016 | 100.0 | 100.0 | 0.0 | 2 | P | P | P |
| 088PP2016 | 96.7 | 100.0 | 0.0 | 6 | P | P | P |
| 089PP2016 | 50.0 | 63.3 | 5.8 | 6 | P | NP | P |
| | 80.0 | 100.0 | 0.0 | 7 | | P | |
| | bd | 96.7 | 5.8 | 8 | | P | |
| 105PP2016 | 95.0 | 100.0 | 0.0 | 2 | P | P | P |
| 106PP2016 | 83.3 | 100.0 | 0.0 | 5 | NP | P | P |
| | 3.3 | 19.9 | 8.7 | 7 | | NP | |
| | bd | 93.3 | 5.8 | 8 | | P | |
| 107PP2016 | 63.0 | 93.3 | 11.5 | 5 | NP | P | P |
| 108PP2016 | 71.7 | 89.8 | 6.3 | 2 | NP | P | P |
| | 0.0 | 3.3 | 5.8 | 7 | | NP | |
| | bd | 90.0 | 17.3 | 8 | | P | |
| 110PP2016 | 83.3 | 100.0 | 0.0 | 3 | NP | P | P |
| 113PP2016 | 76.7 | 100.0 | 0.0 | 5 | P | P | P |
| 114PP2016 | 90.0 | 93.3 | 11.5 | 4 | P | P | P |
| | 3.3 | 3.3 | 5.8 | 7 | | NP | |
| | bd | 100.0 | 0.0 | 8 | | P | |
| 120PP2016 | 27.4 | 52.2 | 13.5 | 3 | NP | NP | NP |
| 123PP2016 | 93.3 | 100.0 | 0.0 | 5 | NP | P | P |
| | 60.7 | 93.0 | 6.1 | 7 | | P | |
| 124PP2016 | 93.3 | 100.0 | 0.0 | 5 | NP | P | P |
| | 65.6 | 100.0 | 0.0 | 7 | | P | |
| 126PP2016 | 86.7 | 100.0 | 0.0 | 5 | NP | P | P |
| | 71.7 | 100.0 | 0.0 | 7 | | P | |
| 127PP2016 | 80.0 | 100.0 | 0.0 | 5 | NP | P | P |
| | 66.7 | 100.0 | 0.0 | 7 | | P | |
| 130PP2017 | 73.3 | 93.3 | 5.8 | 5 | P | P | P |
| 131PP2017 | 82.2 | 100.0 | 0.0 | 5 | P | P | P |
| 134PP2017 | 78.5 | 100.0 | 0.0 | 5 | NP | P | P |
| 137PP2017 | 93.4 | 96.3 | 6.4 | 4 | NP | P | NP |
| | 26.7 | 34.8 | 13.0 | 6 | | NP | |
| | 20.0 | 30.0 | 20.0 | 7 | | NP | |
| 138PP2017 | 60.0 | 85.0 | 8.4 | 2 | NP | NP | NP |
| | bd | 45.9 | 29.3 | 6 | | NP | |
| | bd | 100.0 | 0.0 | 8 | | P | |
| 139PP2017 | 80.7 | 82.6 | 17.7 | 1 | NP | NP | NP |
| | 48.5 | 64.6 | 28.6 | 1 | | NP | |
| | 51.3 | 79.6 | 15.2 | 1 | | NP | |
| | 36.7 | 65.0 | 20.7 | 1 | | NP | |
| | 35.0 | 73.3 | 5.2 | 2 | | NP | |
| 140PP2017 | 16.7 | 34.4 | 15.0 | 5 | NP | NP | NP |
| 141PP2017 | 69.0 | 70.0 | 26.5 | 4 | NP | NP | NP |
| | 23.3 | 26.7 | 5.8 | 6 | | NP | |
| | 14.9 | 49.0 | 11.9 | 7 | | NP | |
| 142PP2017 | 76.7 | 93.3 | 11.5 | 4 | NP | P | P |
| | bd | 90.0 | 10.0 | 8 | | P | |
| 143PP2017 | 80.0 | 96.3 | 6.4 | 4 | NP | P | P |
| 144PP2017 | 91.7 | 100.0 | 0.0 | 2 | NP | P | P |
| 147PP2017 | 50.0 | 66.7 | 15.3 | 5 | NP | NP | NP |
| 149PP2017 | 90.0 | 93.3 | 5.8 | 5 | NP | P | P |
| | 40.0 | 76.7 | 15.3 | 7 | | NP | |
| | bd | 100.0 | 0.0 | 8 | | P | |
| 151PP2017 | 93.4 | 96.3 | 6.4 | 4 | NP | P | P |
| 152PP2017 | 80.0 | 100.0 | 0.0 | 4 | NP | P | P |
| 153PP2017 | 65.0 | 100.0 | 0.0 | 5 | NP | P | P |
| 154PP2017 | 100.0 | 100.0 | 0.0 | 5 | P | P | P |
| | 90.0 | 96.7 | 5.8 | 7 | | P | |
| 155PP2016 | 93.4 | 100.0 | 0.0 | 4 | P | P | P |
| 156PP2017 | 100.0 | 100.0 | 0.0 | 5 | P | P | P |
| 157PP2017 | 100.0 | 100.0 | 0.0 | 6 | P | P | P |
| 158PP2017 | 96.7 | 100.0 | 0.0 | 6 | P | P | P |
| 159PP2017 | 100.0 | 100.0 | 0.0 | 4 | P | P | P |
| B001PP2016 | 100.0 | 100.0 | 0.0 | 4 | P | P | P |
| B002PP2016 | 53.3 | 93.3 | 5.8 | 5 | P | P | P |

| **STRAINS REMOVED FROM THE ANALYSIS** | | | | | | | |
|---|---|---|---|---|---|---|---|
| 019PP2015 | 40.0 | 43.3 | 20.8 | 6 | P | NP | ? (high SD>20% ) |
| | 37.8 | 75.9 | 25.1 | 7 | | NP | |
| | bd | 100.0 | 0.0 | 8 | | P | |
| 027PP2016 | 52.7 | 85.5 | 4.8 | 7 | P | P? | ? (NP/P) |
| | 60.0 | 70.0 | 10.0 | 5 | | NP | |
| | bd | 83.0 | 11.2 | 8 | | NP | |
| 051PP2016 | 62.6 | 75.9 | 5.3 | 3 | P | NP | ? (NP/P) |
| | 66.7 | 90.0 | 10.0 | 6 | | P | |
| | bd | 85.6 | 17.1 | 8 | | P? | |
| 074PP2016 | 72.6 | 83.3 | 20.8 | 3 | P | NP | ? (NP/P) |
| | 74.4 | 100.0 | 0.0 | 7 | | P | |
| 121PP2016 | 100.0 | 100.0 | 0.0 | 6 | NP | P | ? (NP/P) |
| | 51.1 | 82.5 | 10.9 | 7 | | NP | |
| 135PP2017 | 56.7 | 83.3 | 5.8 | 4 | NP | NP | ? (NP/P) |
| | 90.0 | 96.3 | 6.4 | 6 | | P | |
| 145PP2017 | 92.6 | 100.0 | 0.0 | 5 | NP | P | BACTERIAL STOCKS EXCHANGED |
| 146PP2017 | 93.3 | 100.0 | 0.0 | 5 | NP | P | |
| | 57.8 | 86.3 | 15.2 | 7 | | P | |

**Example 3.** Selection of genes allowing for unequivocal determination of pathogenicity of *E. coli* strains.

A discriminant analysis (Linear Discriminant Analysis; LDA) was performed in order to select up to 5 virulence genes, which, either alone or together with a determination of the serotype of the tested strain, would be effective and reliable in determining the pathogenicity of *Escherichia coli* in poultry.

The analysis showed that the best discriminants in the *E. coli* pathogenicity prediction model are genes related to iron metabolism: *iroB*, *iroC*, *iroD*, *iroE*, *iroN*, as well as: *hlyF* (hemolysin), *bor* (prophage lipoprotein) and *ompT* (protease able to cleave colicin), (**Appendix 1** - **LDA analysis all_genes**). In order to avoid a situation in which the prediction model deals with genes of one family, **the following genes were selected for the prediction model: *iroC*, *hlyF*, *ompT*, *bor* and the 078 serotype.**

Finally, in order to ensure high efficiency in predicting strain pathogenicity by the algorithm in each population while maintaining ease of determination, two genes (*iroC, hlyF*) were selected together with the 078 serotype for the algorithm.

**Example 4.** Design of diagnostic test.

### Design

Based on the pathogenicity analysis, a new diagnostic multiplex PCR test was designed to analyze the presence of selected genes responsible for virulence. Table 12 presents the sequences of the original primers for the amplification of selected genes.

The final test to determine the pathogenicity of *E. coli* strains isolated in poultry is as follows: DNA isolated from *E. coli* strains is subjected to multiplex PCR to amplify 2 virulence genes (***iroC*, *hlyF***) and the gene responsible for serotype **078.** Then, the presence of these genes is verified by electrophoresis and the strain is assigned to the appropriate pathogenicity group, i.e. to pathogenic (P) strains - in the presence of at least one of the three genes or to non-pathogenic (NP) strains - in the absence of any of the above genes.

**Table 12. Primers used in diagnostic test**

| **Stage of the test** | **Name** | **Sequence 5'→3'** | **Product size (bp)** |
|---|---|---|---|
| **I stage** | iroC-F (Seq ID No. 1) | ACTATGTGCGCCGTGGTTAT | 732 |
| | iroC-R (Seq ID No. 2) | GTGAACGGGTGTCGATCAGT | |
| | hlyF-F (Seq ID No. 3) | GAGCACCTACTCCACAAGCG | 458 |
| | hlyF-A-R (Seq ID No. 4) | TCGGGCAACCAACAAAGGTA | |
| **II stage** | O78-A-F (Seq ID No. 5) | CACAACTCTCGGCAATATATCATCA | 994 |
| | O78-A-R (Seq ID No. 6) | TATGGGTTTGGTGGTACGTAGT | |

### Verification

The designed diagnostic test in the form of PCR multiplex was performed on all strains from the collection, assigning the strains to appropriate pathogenicity groups P or NP. The obtained results were compared with the results of bioinformatics analysis. The results are summarized in Table 13. it was found that both analyzes are compatible with each other, i.e. strains are assigned to the respective pathogenicity groups in an identical way.

**Table 13. Multiplex PCR results**

| ***E. coli* strain** | PCR products presence | | | Pathogenicity of *E.coli* strains (**NP**- non-pathogenic; **P**-pathogenic) | |
|---|---|---|---|---|---|
| | iroC (732 bp) | hlyF (458 bp) | O78 (994 bp) | Pathogenicity group | SEROTYPE |
| 001PP2015 | - | - | - | **NP** | - |
| 002PP2015 | + | + | - | **P** | - |
| 004PP2015 | + | + | - | **P** | - |
| 005PP2015 | + | + | - | **P** | - |
| 007PP2015 | - | - | - | **NP** | - |
| 009PP2015 | + | + | + | **P** | O78 |
| 011PP2015 | - | - | - | **NP** | - |
| 012PP2015 | + | + | - | **P** | - |
| 014PP2015 | + | + | + | **P** | O78 |
| 015PP2015 | + | + | - | **P** | - |
| 016PP2015 | + | + | + | **P** | O78 |
| 017PP2015 | + | + | - | **P** | - |
| 018PP2015 | + | + | - | **P** | - |
| 019PP2015 | + | + | + | **P** | O78 |
| 020PP2016 | + | + | - | **P** | - |
| 021PP2016 | + | + | - | **P** | - |
| 022PP2016 | + | + | + | **P** | O78 |
| 023PP2016 | + | + | - | **P** | - |
| 024PP2016 | + | + | - | **P** | - |
| 027PP2016 | + | + | - | **P** | - |
| 028PP2016 | + | + | - | **P** | - |
| 029PP2016 | + | + | - | **P** | - |
| 030PP2016 | + | + | - | **P** | - |
| 031 PP2016 | + | + | + | **P** | O78 |
| 032PP2016 | - | - | - | **NP** | - |
| 033PP2016 | + | + | - | **P** | - |
| 034PP2016 | + | + | - | **P** | - |
| 035PP2016 | + | + | + | **P** | O78 |
| 036PP2016 | + | + | - | **P** | - |
| 037PP2016 | + | + | - | **P** | - |
| 038PP2016 | + | + | - | **P** | - |
| 039PP2016 | + | + | + | **P** | O78 |
| 040PP2016 | + | + | + | **P** | O78 |
| 041PP2016 | + | + | + | **P** | O78 |
| 043PP2016 | + | + | + | **P** | O78 |
| 044PP2016 | + | + | + | **P** | O78 |
| 045PP2016 | + | + | - | **P** | - |
| 046PP2016 | + | + | - | **P** | - |
| 047PP2016 | - | - | - | **NP** | - |
| 048PP2016 | - | - | - | **NP** | - |
| 049PP2016 | - | - | - | **NP** | - |
| 050PP2016 | + | + | - | **P** | - |
| 051PP2016 | - | - | - | **NP** | - |
| 052PP2016 | - | - | - | **NP** | - |
| 053PP2016 | + | + | - | **P** | - |
| 054PP2016 | + | + | - | **P** | - |
| 057PP2016 | + | + | - | **P** | - |
| 059PP2016 | + | + | - | **P** | - |
| 062PP2016 | + | + | - | **P** | - |
| 063PP2016 | + | + | - | **P** | - |
| 065PP2016 | - | - | - | **NP** | - |
| 066PP2016 | + | + | - | **P** | - |
| 067PP2016 | + | + | - | **P** | - |
| 069PP2016 | + | + | - | **P** | - |
| 070PP2016 | + | + | - | **P** | - |
| 073PP2016 | + | + | - | **P** | - |
| 074PP2016 | + | + | - | **P** | - |
| 075PP2016 | + | + | - | **P** | - |
| 076PP2016 | + | + | + | **P** | O78 |
| 077PP2016 | + | + | - | **P** | - |
| 078PP2016 | + | + | - | **P** | - |
| 079PP2016 | + | + | - | **P** | - |
| 08OPP2016 | + | + | - | **P** | - |
| 081PP2016 | + | + | - | **P** | - |
| 082PP2016 | - | - | - | **NP** | - |
| 083PP2016 | + | + | + | **P** | O78 |
| 084PP2016 | + | + | - | **P** | - |
| 085PP2016 | + | + | - | **P** | - |
| 086PP2016 | + | + | - | **P** | - |
| 087PP2016 | + | + | + | **P** | O78 |
| 088PP2016 | + | + | + | **P** | O78 |
| 089PP2016 | + | + | - | **P** | - |
| ß_001PP2016 | + | + | - | **P** | - |
| ß_002PP2016 | + | + | - | **P** | - |
| 105PP2016 | + | + | - | **P** | - |
| 106PP2016 | + | + | - | **P** | - |
| 107PP2016 | + | + | - | **P** | - |
| 108PP2016 | - | - | - | **NP** | - |
| 110PP2016 | + | + | - | **P** | - |
| 113PP2016 | + | + | - | **P** | - |
| 114PP2016 | + | + | - | **P** | - |
| 120PP2016 | - | + | - | **P** | - |
| 121PP2016 | + | + | - | **P** | - |
| 123PP2016 | + | + | - | **P** | - |
| 124PP2016 | + | + | - | **P** | - |
| 126PP2016 | - | + | - | **P** | - |
| 127PP2016 | - | + | - | **P** | - |
| 130PP2017 | + | + | - | **P** | - |
| 131PP2017 | + | + | - | **P** | - |
| 134PP2017 | + | + | - | **P** | - |
| 135PP2017 | + | + | - | **P** | - |
| 137PP2017 | - | - | - | **NP** | - |
| 138PP2017 | - | - | - | **NP** | - |
| 139PP2017 | - | - | - | **NP** | - |
| 140PP2017 | - | - | - | **NP** | - |
| 141PP2017 | + | + | - | **P** | - |
| 142PP2017 | + | + | - | **P** | - |
| 143PP2017 | + | + | - | **P** | - |
| 144PP2017 | - | - | + | **P** | O78 |
| 147PP2017 | - | - | - | **NP** | - |
| 149PP2017 | - | - | - | **NP** | - |
| 151PP2017 | - | - | + | **P** | O78 |
| 152PP2017 | - | - | + | **P** | O78 |
| 153PP2017 | + | + | - | **P** | - |
| 154PP2017 | + | + | - | **P** | - |
| 155PP2017 | + | + | - | **P** | - |
| 156PP2017 | + | + | + | **P** | O78 |
| 157PP2017 | + | + | - | **P** | - |
| 158PP2017 | + | + | + | **P** | O78 |
| 159PP2017 | + | + | - | **P** | - |
| K12 | - | - | - | **NP** | - |

### Evaluation of the effectiveness of the test

Based on the conducted experiments, the diagnostic method identifying *E. coli* strains as pathogenic for poultry (APEC) has been shown to be highly effective - the test allows to assess the strain as pathogenic or non-pathogenic with an accuracy of 97.7% and 94.1%, respectively.

### References

Antao, EM., Ganwu, L., Wieler, L., Preisinger, R., Ewers, C. Identification and characterization of a novel avian pathogenic E. coli (APEC) fimbrial adhesion. EP2298798B1.
Barbieri, N. L., de Oliveira, A. L., Tejkowski, T. M., Pavanelo, D. B., Rocha, D. A., Matter, L. B., ... Horn, F. (2013). Genotypes and pathogenicity of cellulitis isolates reveal traits that modulate APEC virulence. PloS one, 8(8), e72322. doi:10.1371/journal.pone.0072322.
Barnes, H.J., Nolan, L.K., Vaillancourt, JP. (2008). Colibacillosis in Diseases of Poultry, 12th Edition, Blackwell Publishing.
Dissanayake, DR., Octavia, S., Lan, R. (2014). Population structure and virulence content of avian pathogenic Escherichia coli isolated from outbreaks in Sri Lanka. Veterinary Microbiology 168, 403-412; doi: 10.1016/j.vetmic.2013.11.028.
Dziva, F., Stevens, MP. (2008). Colibacillosis in poultry: unravelling the molecular basis of virulence of avian pathogenic Escherichia coli in their natural hosts. Avian Pathol. 2008 Aug;37(4):355-66. doi: 10.1080/03079450802216652.
Dziva, F., Hauser, H., Connor, T. R., van Diemen, P. M., Prescott, G., Langridge, G. C., ... Stevens, M. P. (2013). Sequencing and functional annotation of avian pathogenic Escherichia coli serogroup 078 strains reveal the evolution of E. coli lineages pathogenic for poultry via distinct mechanisms. Infection and immunity, 81(3), 838-849. doi:10.1128/IAI.00585-12.
Ewers, C., Janssen, T., Kiessling, S., Philipp, H.C., Wieler, L.H. (2005). Rapid detection of virulence-associated genes in avian pathogenic Escherichia coli by multiplex polymerase chain reaction. Avian Dis. Jun; 49(2): 269-73. doi: 10.1637/7293-102604R
Ewers, C., Antão, E. M., Diehl, I., Philipp, H. C., & Wieler, L. H. (2008). Intestine and environment of the chicken as reservoirs for extraintestinal pathogenic Escherichia coli strains with zoonotic potential. Applied and environmental microbiology, 75(1), 184-192. doi:10.1128/AEM.01324-08
Guabiraba, R., Schouler, C. (2015). Avian colibacillosis: still many black holes. FEMS Microbiology Letters, 362, fnv118; doi: 10.1093/femsle/fnv118.
Huja, S., Oren, Y., Trost, E., Brzuszkiewicz, E., Biran, D., Blom, J., ... Dobrindt, U. (2015). Genomic avenue to avian colisepticemia. mBio, 6(1), e01681-14. doi:10.1128/mBio.01681-14.
Hussein, AH., Ghanem, IA., Eid, AA., Ali, MA., Sherwood, JS., Li, G., Nolan, LK., Logue, CM. (2013). Molecular and phenotypic characterization of Escherichia coli isolated from broiler chicken flocks in Egypt. Avian Dis. 57(3): 602-11. doi: 10.1637/10503-012513-Reg.1
Iguchi, A., Iyoda, S., Seto, K., Morita-Ishihara, T., Scheutz, F., Ohnishi, M., & Pathogenic E. coli Working Group in Japan (2015). Escherichia coli O-Genotyping PCR: a Comprehensive and Practical Platform for Molecular O Serogrouping. Journal of clinical microbiology, 53(8), 2427-2432. doi:10.1128/JCM.00321-15.
Janßen, T., Dr Hans, C. P., Voss, M., Preisinger, R., Lotha,r H., Wieler, L. (2003). Multiplex PCR is the first technique to allow the specific and sensitive detection of avian pathogenic Escherichia coli (APEC). LOHMANN Information, 28/2003, 1-5.
Joensen, K. G., Tetzschner, A.M., Iguchi, A., Aarestrup, F.M. and Scheutz, F. (2015). Rapid and easy in silico serotyping of Escherichia coli using whole genome sequencing (WGS) data. J.Clin.Microbiol. 53(8):2410-2426. doi:JCM.00008-15 [pii];10.1128/JCM.00008-15 [doi]
Johnson, T. J., Wannemuehler, Y., Doetkott, C., Johnson, S. J., Rosenberger, S. C., & Nolan, L. K. (2008). Identification of minimal predictors of avian pathogenic Escherichia coli virulence for use as a rapid diagnostic tool. Journal of clinical microbiology, 46(12), 3987-3996. doi:10.1128/JCM.00816-08.
Jørgensen, S. L., Kudirkiene, E., Li, L., Christensen, J. P., Olsen, J. E., Nolan, L., & Olsen, R. H. (2017). Chromosomal features of Escherichia coli serotype 02:K2, an avian pathogenic E. coli. Standards in genomic sciences, 12, 33. doi:10.1186/s40793-017-0245-3.
Krawczyk, B., Samet, A., Leibner, J., Sledziriska, A., & Kur, J. (2006). Evaluation of a PCR melting profile technique for bacterial strain differentiation. Journal of clinical microbiology, 44(7), 2327-2332. doi:10.1128/JCM.00052-06.
Lee, SY., Yoo, SM., Chang, KH., Yoo, SY., Yo, NCh., Yoo, WM., Keum, KCh., Kim, JM., Lee, G. Nucleic acid probes for detection of non-viral organisms. US 2007/0065817 A1.
Lee, SY., Yoo, SM., Shin, SY., Keum, KCh., Yoo, NCh., Yoo, WM., Kim, JM., Choi, JY. DNA chip for detection of Escherichia coli. WO2008/084888 A.
Lindstedt, B. A., Finton, M. D., Porcellato, D., & Brandal, L. T. (2018). High frequency of hybrid Escherichia coli strains with combined Intestinal Pathogenic Escherichia coli (IPEC) and Extraintestinal Pathogenic Escherichia coli (ExPEC) virulence factors isolated from human faecal samples. BMC infectious diseases, 18(1), 544. doi:10.1186/s12879-018-3449-2.
Lutful Kabir S. M. (2010). Avian colibacillosis and salmonellosis: a closer look at epidemiology, pathogenesis, diagnosis, control and public health concerns. International journal of environmental research and public health, 7(1), 89-114. doi:10.3390/ijerph7010089.
Mellata M. (2013). Human and avian extraintestinal pathogenic Escherichia coli: infections, zoonotic risks, and antibiotic resistance trends. Foodborne pathogens and disease, 10(11), 916-932. doi:10.1089/fpd.2013.1533.
Paixão, AC., Ferreira, AC., Fontes, M., Themudo, P., Albuquerque, T., Soares, MC., Fevereiro, M., Martins, L., Corrêa de Sá, MI. (2016). Detection of virulence-associated genes in pathogenic and commensal avian Escherichia coli isolates. Poultry Science 95:1646-1652. doi: 10.3382/ps/pew087
Roussan, D.A., Zakaria, H., Khawaldeh, G., Shaheen, I. (2014). Differentiation of avian pathogenic Escherichia coli strains from broiler chickens by multiplex polymerase chain reaction (PCR) and random amplified polymorphic DNA (RAPD) Open J. Vet. Med. 4: 211-219. doi: 10.4236/ojvm.2014.410025.
Sarowska, J., Futoma-Koloch, B., Jama-Kmiecik, A., Frej-Madrzak, M., Ksiazczyk, M., Bugla-Ploskonska, G., & Choroszy-Krol, I. (2019). Virulence factors, prevalence and potential transmission of extraintestinal pathogenic Escherichia coli isolated from different sources: recent reports. Gut pathogens, 11, 10. doi:10.1186/s13099-019-0290-0.
Schouler, C., Schaeffer, B., Brée, A., Mora, A., Dahbi, G., Biet, F., ... Moulin-Schouleur, M. (2012). Diagnostic strategy for identifying avian pathogenic Escherichia coli based on four patterns of virulence genes. Journal of clinical microbiology, 50(5), 1673-1678. doi:10.1128/JCM.05057-11.
Silveira, F., Maluta, RP., Tiba, MR., de Paiva, JB., Guastalli, EA., da Silveira, WD. (2016). Comparison between avian pathogenic (APEC) and avian faecal (AFEC) Escherichia coli isolated from different regions in Brazil. The Veterinary Journal 217, 65-67; doi: 10.1016/j.tvjl.2016.06.007.
Skyberg, JA., Horne, SM., Giddings, CW., Wooley, RE., Gibbs, PS., Nolan, LK. (2003). Characterizing avian Escherichia coli isolates with multiplex polymerase chain reaction. Avian Dis. Oct-Dec; 47(4): 1441-7. doi: 10.1637/7030.
Sokurenko, E., Johnson, J.R., Weissman, S., Tchesnokova, V. High-Resolution Clonal Typing of Escherichia coli. US 2014/0193824 A1.
Stromberg, Z. R., Johnson, J. R., Fairbrother, J. M., Kilbourne, J., Van Goor, A., Curtiss, R., Rd, & Mellata, M. (2017). Evaluation of Escherichia coli isolates from healthy chickens to determine their potential risk to poultry and human health. PloS one, 12(7), e0180599. doi:10.1371/journal.pone.0180599.
Subedi, M., Luitel, H., Devkota, B., Bhattarai, R. K., Phuyal, S., Panthi, P., ... Chaudhary, D. K. (2018). Antibiotic resistance pattern and virulence genes content in avian pathogenic Escherichia coli (APEC) from broiler chickens in Chitwan, Nepal. BMC veterinary research, 14(1), 113. doi:10.1186/s12917-018-1442-z.
van der Westhuizen, WA., Bragg, RR. (2012). Multiplex polymerase chain reaction for screening avian pathogenic Escherichia coli for virulence genes. Avian Pathol. 41(1): 33-40. doi: 10.1080/03079457.2011.631982.
Weigel, LM., Tenover, FC. Oligonucleotide probes for detecting Enterobacteraceae and quinolone-resistant Enterobacteraceae. US 2008/0199877 A1.

## Claims

1. A method of identification of *E. coli* pathogenic to birds (APEC), **characterized in that**:
a) DNA is isolated from the tested sample of biological material,
b) in the sample of isolated DNA, the presence of virulence genes: *iroC* and *hlyF* and the gene encoding the serotype 078 are checked,
c) the presence of at least one gene among virulence genes or the gene encoding the O78 serotype is the indicative of the APEC strain in the tested sample.

2. The method according to claim 1, **characterized in that** in step a) the test sample is a tissue sample of a sick bird or an environmental sample.

3. The method according to claim 1, **characterized in that** in step b) the sample of the isolated DNA contains bacterial genomic DNA, in particular *E. coli* DNA.

4. The method according to claim 1, **characterized in that** in step b) the presence of mentioned genes is checked by PCR.

5. The method according to claim 1 or 4, **characterized in that** in step b) it uses the multiplex PCR method and the set of primers shown as Seq. ID No. 1-6, where:
- the presence of the *iroC* virulence gene is indicated by the presence of a product with a length of 732 bp,
- the presence of the *hlyF* virulence gene is indicated by the presence of a product with a length of 458 bp, while
- the presence of the gene encoding the antigen of serotype 078 is indicated by the presence of a product with a length of 994 bp.

6. The method according to claim 2, **characterized in that** identifying the APEC strain means confirmation of colibacillosis in a sick bird, especially breeding poultry, preferably chickens.

7. The method according to claim 3, **characterized in that** non-detection of any of the mentioned genes indicates infection with a non-pathogenic strain.

8. A kit for detection of Avian Pathogenic *Escherichia coli* (APEC), **characterized in that** it contains:
- an oligonucleotide containing at least 20 consecutive nucleotides belonging to the iroCvirulence gene shown in Seq. ID No. 7,
- an oligonucleotide comprising at least 20 consecutive nucleotides belonging to the *hlyF* virulence gene shown in Seq. ID No. 8,
- an oligonucleotide containing at least 20 consecutive nucleotides belonging to the gene encoding the antigen of serotype 078 shown in Seq. ID No. 9.

9. The kit according to claim 8, **characterized in that** it contains oligonucleotides with the sequences shown as Seq. ID No. 1-6.

10. Use *in vitro* of the according to claim 8, in a diagnosis of colibacillosis in birds, in particular breeding poultry, preferably chickens.

11. Use *in vitro* of the kit including:
- an oligonucleotide containing at least 20 consecutive nucleotides belonging to the *iroC* virulence gene shown in Seq. ID No. 7,
- an oligonucleotide comprising at least 20 consecutive nucleotides belonging to the *hlyF* virulence gene shown in Seq. ID No. 8,
- an oligonucleotide containing at least 20 consecutive nucleotides belonging to the gene encoding the antigen of serotype 078 shown in Seq. ID No. 9,
for determining the degree of pathogenicity of *E. coli* strain for birds.

12. The use of the kit according to claim 10, **characterized in that** the kit contains oligonucleotides with the sequences shown as Seq. ID No. 1-6 .

## Patentansprüche

1. Verfahren zur Erkennung von für Vögel pathogenes *E. coli* (APEC), **dadurch gekennzeichnet, dass**:
a) DNA aus der getesteten Probe von biologischem Material isoliert wird,
b) in der Probe aus isolierter DNA auf das Vorhandensein von Virulenzgenen: *iroC* und *hlyF* und das Gen, das den Serotyp 078 codiert, getestet wird,
c) das Vorhandensein mindestens eines Genes von Virulenzgenen oder dem Gen, das den O78-Serotyp codiert, der Hinweis darauf ist, dass der APEC-Stamm in der getesteten Probe ist.

2. Verfahren nach Anspruch 1, **dadurch gekennzeichnet, dass** im Schritt a) die Testprobe eine Gewebeprobe eines kranken Vogels oder eine Umweltprobe ist.

3. Verfahren nach Anspruch 1, **dadurch gekennzeichnet, dass** im Schritt b) die Probe der isolierten DNA bakterielle Genom-DNA, insbesondere *E. coli*-DNA, enthält.

4. Verfahren nach Anspruch 1, **dadurch gekennzeichnet, dass** in Schritt b) auf das Vorhandensein genannter Gene durch PCR getestet wird.

5. Verfahren nach Anspruch 1 oder 4, **dadurch gekennzeichnet, dass** im Schritt b) das Multiplex-PCR-Verfahren und die Gruppe von Primern, die als Seq. ID Nr. 1-6 gezeigt sind, verwendet werden, wobei:
- das Vorhandensein des *iroC*-Virulenzgens durch das Vorhandensein eines Produkts mit einer Länge von 732 bp gekennzeichnet wird,
- das Vorhandensein des *hlyF*-Virulenzgens durch das Vorhandensein eines Produkts mit einer Länge von 458 bp gekennzeichnet wird, wohingegen
- das Vorhandensein des Gens, das das Antigen des Serotyps 078 codiert, durch das Vorhandensein eines Produkts mit einer Länge von 994 bp gekennzeichnet wird.

6. Verfahren nach Anspruch 2, **dadurch gekennzeichnet, dass** Erkennen des APEC-Stamms eine Bestätigung von Colibacillosis in einem kranken Vogel, insbesondere in Brutgeflügel, bevorzugt Hühnerküken, bedeutet.

7. Verfahren nach Anspruch 3, **dadurch gekennzeichnet, dass** eine Nichterkennung eines der genannten Gene eine Infektion mit einem nicht-pathogenen Stamm kennzeichnet.

8. Kit zur Erkennung von avianpathogenem *Escherichia coli* (APEC), **dadurch gekennzeichnet, dass** das Kit enthält:
- ein Oligonucleotid, das mindestens 20 aufeinanderfolgende Nucleotide enthält, die zu dem *iroC*-Virulenzgen gehören, das in Seq. ID Nr. 7 gezeigt ist,
- ein Oligonucleotid mit mindestens 20 aufeinanderfolgenden Nucleotiden, die zu dem in Seq. ID Nr. 8 gezeigten *hlyF*-Virulenzgen gehören,
- ein Oligonucleotid, das mindestens 20 aufeinanderfolgende Nucleotide enthält, die zu dem Gen gehören, das in Seq. ID Nr. 9 gezeigte Antigen vom Serotyp 078 codiert.

9. Kit nach Anspruch 8, **dadurch gekennzeichnet, dass** es Oligonucleotide mit Sequenzen enthält, die als Seq. ID Nr. 1-6 gezeigt sind.

10. *In*-*vitro*-Verwendung des Kits nach Anspruch 8 in einer Diagnose von Colibacillosis in Vögeln, insbesondere in Brutgeflügel, bevorzugt im Huhn.

11. *In*-*vitro*-Verwendung des Kits, der enthält:
- ein Oligonucleotid, das mindestens 20 aufeinanderfolgende Nucleotide enthält, die zu dem *iroC*-Virulenzgen gehören, das in Seq. ID Nr. 7 gezeigt ist,
- ein Oligonucleotid mit mindestens 20 aufeinanderfolgenden Nucleotiden, die zu dem in Seq. ID Nr. 8 gezeigten *hlyF*-Virulenzgen gehören,
- ein Oligonucleotid, das mindestens 20 aufeinanderfolgende Nucleotide enthält, die zu dem Gen gehören, das das in Seq. ID Nr. 9 gezeigte Antigen des Serotyps 078 codiert,
zur Ermittlung des Grades an Pathogenizität des *E. coli*-Stamm für Vögel.

12. Verwendung des Kits nach Anspruch 10, **dadurch gekennzeichnet, dass** das Kit Oligonucleotide mit den Sequenzen, die als Seq. ID Nr. 1-6 gezeigt sind, enthält.

## Revendications

1. Méthode d'identification d'*E*. *coli* pathogène pour les oiseaux (APEC), **caractérisée en ce que** :
a) de l'ADN est isolé à partir de l'échantillon testé de matière biologique,
b) dans l'échantillon d'ADN isolé, la présence de gènes de virulence : *iroC* et *hlyF* et du gène codant le sérotype 078 est vérifiée,
c) la présence d'au moins un gène parmi des gènes de virulence ou le gène codant le sérotype 078 est l'indication de la souche APEC dans l'échantillon testé.

2. Méthode selon la revendication 1, **caractérisée en ce qu'**à l'étape a) l'échantillon testé est un échantillon de tissu d'un oiseau malade ou un échantillon environnemental.

3. Méthode selon la revendication 1, **caractérisée en ce qu'**à l'étape b) l'échantillon de l'ADN isolé contient de l'ADN génomique bactérien, en particulier de l'ADN d'*E*. *coli.*

4. Méthode selon la revendication 1, **caractérisée en ce qu'**à l'étape b) la présence des gènes mentionnés est vérifiée par PCR.

5. Méthode selon la revendication 1 ou 4, **caractérisée en ce qu'**à l'étape b), on utilise la méthode PCR multiplex et l'ensemble d'amorces montrées comme Seq. ID N° 1 à 6, où :
- la présence du gène de virulence *iroC* est indiquée par la présence d'un produit d'une longueur de 732 pb,
- la présence du gène de virulence *hlyF* est indiquée par la présence d'un produit d'une longueur de 458 pb, tandis que
- la présence du gène codant l'antigène du sérotype 078 est indiquée par la présence d'un produit d'une longueur de 994 pb.

6. Méthode selon la revendication 2, **caractérisée en ce que** l'identification de la souche APEC signifie la confirmation de la colibacillose chez un oiseau malade, notamment les volailles de reproduction, préférentiellement les poulets.

7. Méthode selon la revendication 3, **caractérisée en ce que** la non-détection de l'un quelconque des gènes mentionnés indique une infection par une souche non pathogène.

8. Kit pour la détection d'*Escherichia coli* pathogène aviaire (APEC), **caractérisé en ce qu'**il contient :
- un oligonucléotide contenant au moins 20 nucléotides consécutifs appartenant au gène de virulence *iroC* montré dans Seq. ID N° 7,
- un oligonucléotide comprenant au moins 20 nucléotides consécutifs appartenant au gène de virulence *hlyF* montré dans Seq. ID N° 8,
- un oligonucléotide contenant au moins 20 nucléotides consécutifs appartenant au gène codant l'antigène du sérotype 078 montré dans Seq. ID N° 9.

9. Kit selon la revendication 8, **caractérisé en ce qu'**il contient des oligonucléotides ayant les séquences montrées comme Seq. ID N° 1 à 6.

10. Utilisation *in vitro* du kit selon la revendication 8, dans un diagnostic de colibacillose chez les oiseaux, en particulier les volailles de reproduction, préférentiellement les poulets.

11. Utilisation *in vitro* du nécessaire incluant :
- un oligonucléotide contenant au moins 20 nucléotides consécutifs appartenant au gène de virulence *iroC* montré dans Seq. ID n° 7,
- un oligonucléotide comprenant au moins 20 nucléotides consécutifs appartenant au gène de virulence *hlyF* montré dans Seq. ID n° 8,
- un oligonucléotide contenant au moins 20 nucléotides consécutifs appartenant au gène codant l'antigène du sérotype 078 montré dans Seq. ID n° 9,
pour déterminer le degré de pathogénicité d'une souche d'*E*. *coli* pour les oiseaux.

12. Utilisation du kit selon la revendication 10, **caractérisée en ce que** le nécessaire contient des oligonucléotides ayant les séquences montrées comme Seq. ID n° 1 à 6.
